# EUROPEAN PATENT SPECIFICATION

(11) **EP 1 937 810 B1**
(45) Date of publication and mention of the grant of the patent: **21.11.2012**
(21) Application number: 06815190.1
(22) Date of filing: 22.09.2006
(51) Int. Cl.: C12N 15/00, C12N 1/20, C12N 7/00

(54) **DNA MODULAR CLONING VECTOR PLASMIDS AND METHODS FOR THEIR USE**
DNA-VEKTORPLASMIDE FÜR MODULARE KLONIERUNG SOWIE VERFAHREN ZU DEREN VERWENDUNG
PLASMIDES DE VECTEURS DE CLONAGE MODULAIRE D'ADN ET LEURS MÉTHODES D'UTILISATION

(30) Priority: 22.09.2005 US 233246
(43) Date of publication of application: 02.07.2008
(60) Divisional application: 11157471.1
(73) Proprietor: Intrexon Corporation, Blacksburg, VA 24060 (US)
(72) Inventor: ZHOU, Jiang, F., Dollard-des-Ormeaux, Québec H9A 3B1 (CA); REED, Thomas, Blacksburg, Virginia 24060 (US)
(74) Representative: Clements, Andrew Russell Niel
(86) International application number: PCT/US2006/036992
(87) International publication number: WO 2007/038276

(56) References cited:
- US-A- 5 061 628
- US-A- 5 192 676
- US-A- 5 787 104
- US-A1- 2004 185 556
- US-A1- 2005 176 099
- US-B1- 6 245 545
- ZABAROVSKA ET AL.: 'Notl passporting to identify species composition of complex microbial systems' NUCLEIC ACIDS RES. vol. 31, no. 2, January 2003, page E5-5, XP008126493
- BRAY ET AL.: 'Physical linkage of the genes for platelet membrane glycoproteins IIb and IIIa' PROC. NATL. ACAD. SCI. U S A vol. 85, no. 22, November 1988, pages 8683 - 8687, XP008126502

## Description

### CROSS-REFERENCE TO RELATED APPLICATIONS

This application is a continuation-in-part, and claims the benefit under 35 U.S.C. 120 of Application No.10/682,764, filed October 9, 2003, which claims the benefit under 35 U.S.C. 119(e) of provisional application 60/417,282, filed October 9, 2002.

### FIELD OF INVENTION

The present invention relates to the field of cloning vector plasmids, and to the use of cloning vector plasmids to build DNA constructs or transgenes.

### BACKGROUND OF THE INVENTION

The foundation of molecular biology is recombinant DNA technology, which can here be summarized as the modification and propagation of nucleic acids for the purpose of studying the structure and function of the nucleic acids and their protein products.

Individual genes, gene regulatory regions, subsets of genes, and indeed entire chromosomes in which they are contained, are all comprised of double-stranded anti-parallel sequences of the nucleotides adenine, thymine, guanine and cytosine, identified conventionally by the initials A, T, G, and C, respectively. These DNA sequences, as well as cDNA sequences, which are double stranded DNA copies derived from mRNA (messenger RNA) molecules, can be cleaved into distinct fragments, isolated, and inserted into a vector such as a bacterial plasmid to study the gene products. A plasmid is an extra-chromosomal piece of DNA that was originally derived from bacteria, and can be manipulated and reintroduced into a host bacterium for the purpose of study or production of a gene product. The DNA of a plasmid is similar to all chromosomal DNA, in that it is composed of the same A, T, G, and C nucleotides encoding genes and gene regulatory regions, however, it is a relatively small molecule comprised of less than approximately 30,000 base-pairs, or 30 kilobases (kb). In addition, the nucleotide base pairs of a double-stranded plasmid form a continuous circular molecule, also distinguishing plasmid DNA from that of chromosomal DNA.

Plasmids enhance the rapid exchange of genetic material between bacterial organisms and allow rapid adaptation to changes in environment, such as temperature, food supply, or other challenges. Any plasmid acquired must express a gene or genes that contribute to the survival of the host or else it will be destroyed or discarded by the organism since the maintenance of unnecessary plasmids would be a wasteful use of resources. A clonal population of cells contains identical genetic material, including any plasmids it might harbor. Use of a cloning vector plasmid with a DNA insert in such a clonal population of host cells will amplify the amount of the DNA of interest available. The DNA so cloned may then be isolated and recovered for subsequent manipulation in the steps required for building a DNA construct. Thus, it can be appreciated that cloning vector plasmids are useful tools in the study of gene function, providing the ability to rapidly produce large amounts of the DNA insert of interest.

While some elements found in plasmids are naturally occurring, others have been engineered to enhance the usefulness of plasmids as DNA vectors. These include antibiotic- or chemical-resistance genes and a multiple cloning site (MCS), among others. Each of these elements has a role in the present invention, as well as in the prior art. Description of the role each element plays will highlight the limitations of the prior art and demonstrate the utility of the present invention.

A particularly useful plasmid-born gene that can be acquired by a host is one that would confer antibiotic resistance. In the daily practice of recombinant DNA technology, antibiotic resistance genes are exploited as positive or negative selection elements to preferentially enhance the culture and amplification of the desired plasmid over that of other plasmids.

In order to be maintained by a host bacterium a plasmid must also contain a segment of sequences that direct the host to duplicate the plasmid. Sequences known as the origin of replication (ORI) element direct the host to use its cellular enzymes to make copies of the plasmid. When such a bacterium divides, the daughter cells will each retain a copy or copies of any such plasmid. Certain strains of E. coli bacteria have been derived to maximize this duplication, producing upwards of 300 copies per bacterium. In this manner, the cultivation of a desired plasmid can be enhanced.

Another essential element in any cloning vector is a location for insertion of the genetic materials of interest. This is a synthetic element that has been engineered into "wild type" plasmids, thus conferring utility as a cloning vector. Any typical commercially-available cloning vector plasmid contains at least one such region, known as a multiple cloning site (MCS). A MCS typically comprises nucleotide sequences that may be cleaved by a single or a series of restriction endonuclease enzymes (hereafter referred to as "restriction enzymes"), each of which has a distinct endonuclease restriction site and cleavage pattern. These endonuclease sites, or endonuclease restriction sites (hereafter referred to as "restriction sites"), encoded in the DNA molecule typically comprise a double-stranded palindromic sequence. For some restriction enzymes, as few as 4-6 nucleotides are sufficient to provide a restriction site, while some restriction enzymes require a restriction site of 8 or more nucleotides. The enzyme EcoR1 for example, recognizes the hexanucleotide sequence: ^{5'}G-A-A-T-T-C^{3'}, wherein 5' indicates the end of the molecule known by convention as the "upstream" end, and 3' likewise indicates the "downstream" end. The complementary strand of the restriction site would be its anti-parallel strand, ^{3'}G-A-A-T-T-C- ^{5'}. Thus the double stranded restriction site can be represented within the larger double-stranded molecule in which it occurs as:
^{5'}...... G-A-A-T-T-C ......^{3'}
^{3'}...... C-T-T-A-A-G ...... ^{5'}

Like many other restriction enzymes, EcoR1 does not cleave exactly at the axis of dyad symmetry, but at positions four nucleotides apart in the two DNA strands between the nucleotides indicated by a "/":
^{5'} ...... G/A-A-T-T-C .......^{3'}
^{3'}...... C-T-T-A-A/G ....... ^{3'}
such that double-stranded DNA molecule is cleaved and has the resultant configuration of nucleotides at the newly formed "ends":
^{5'}....... G ^{3'} 5' A-A-T-T-C ....... ^{3'}
^{3'}......C-T-T-A-A ^{5' 3'} G....... ^{5'}

This staggered cleavage yields fragments of DNA with protruding 5' termini. Because A-T and G-C pairs are spontaneously formed when in proximity with each other, protruding ends such as these are called cohesive or "sticky" ends. Any one of these termini can form hydrogen bonds with any other complementary termini cleaved with the same restriction enzyme. Since any DNA that contains a specific restriction site will be cut in the same manner as any other DNA containing the same sequence, those cleaved ends will be complementary. Therefore, the ends of any DNA molecules cut with the same restriction enzyme "match" each other in the way adjacent pieces of a jigsaw puzzle "match," and can be enzymatically linked together. It is this property that permits the formation of recombinant DNA molecules, and allows the introduction of foreign DNA fragments into bacterial plasmids, or into any other DNA molecule.

A further general principle to consider when building recombinant DNA molecules is that all restriction sites occurring within a molecule will be cut with a particular restriction enzyme, not just the site of interest. The larger a DNA molecule, the more likely it is that any restriction site will reoccur. Assuming that any restriction sites are distributed randomly along a DNA molecule, a tetranucleotide site will occur, on the average, once every 4⁴ (i.e., 256) nucleotides, whereas a hexanucleotide site will occur once every 4⁶ (i.e., 4096) nucleotides, and octanucleotide sites will occur once every 4⁸ (i.e., 114,688) nucleotides. Thus, it can be readily appreciated that shorter restriction sites will occur frequently, while longer ones will occur rarely. When planning the construction of a transgene or other recombinant DNA molecule, this is a vital issue, since such a project frequently requires the assembly of several pieces of DNA of varying sizes. The larger these pieces are, the more likely that the sites one wishes to use occur in several pieces of the DNA components, making manipulation difficult, at best.

Frequently occurring restriction sites are herein referred to as common restriction sites, and the endonucleases that cleave these sites are referred to as common restriction enzymes. Restriction enzymes with cognate sequences greater than 6 nucleotides are referred to as rare restriction enzymes, and their cognate sites as rare restriction sites. However, there are some restriction sites of 6 bp that occur more infrequently than would be statistically predicted, and these sites and the endonucleases that cleave them are also referred to as rare. Thus, the designations "rare" and "common" do not refer to the relative abundance or availability of any particular restriction enzyme, but rather to the frequency of occurrence of the sequence of nucleotides that make up its cognate restriction site within any DNA molecule or isolated fragment of a DNA molecule, or any gene or its DNA sequence.

A second class of restriction enzymes has recently been isolated, called homing endonuclease (HE) enzymes. HE enzymes have large, asymmetric restriction sites (12-40 base pairs). HE restriction sites are extremely rare. For example, the HE known as I-SceI has an 18 bp restriction site (5'...TAGGGATAACAGGGTAAT...3'), predicted to occur only once in every 7x10¹⁰ base pairs of random sequence. This rate of occurrence is equivalent to only one site in 20 mammalian-sized genomes. The rare nature of HE sites greatly increases the likelihood that a genetic engineer can cut a final transgene product without disrupting the integrity of the transgene if HE sites were included in appropriate locations in a cloning vector plasmid.

Since a DNA molecule from any source organism will be cut in identical fashion by its cognate restriction enzyme, foreign pieces of DNA from any species can be cut with a restriction enzyme, inserted into a bacterial plasmid vector that was cleaved with the same restriction enzyme, and amplified in a suitable host cell. For example, a human gene may be cut in 2 places with a restriction enzyme known as EcoR1, and the desired fragment with EcoR1 ends can be isolated and mixed with a plasmid that was also cut with EcoR1 in what is commonly known as a ligation reaction or ligation mixture. Under the appropriate conditions in the ligation mixture, some of the isolated human gene fragments will match up with the ends of the plasmid molecules. These newly joined ends can link together and enzymatically recircularize the plasmid, now containing its new DNA insert. The ligation mixture is then introduced into *E*. *coli* or another suitable host, and the newly engineered plasmids will be amplified as the bacteria divide. In this manner, a relatively large number of copies of the human gene may be obtained and harvested from the bacteria. These gene copies can then be further manipulated for the purpose of research, analysis, or production of its gene product protein.

Recombinant DNA technology is frequently embodied in the generation of so-called "transgenes." Transgenes frequently comprise a variety of genetic materials that are derived from one or more donor organisms and introduced into a host organism. Typically, a transgene is constructed using a cloning vector as the starting point or "backbone" of the project, and a series of complex cloning steps are planned to assemble the final product within that vector. Elements of a transgene, comprising nucleotide sequences, include, but are not limited to 1) regulatory promoter and/or enhancer elements, 2) a gene that will be expressed as a mRNA molecule, 3) DNA elements that provide mRNA message stabilization, 4) nucleotide sequences mimicking mammalian intronic gene regions, and 5) signals for mRNA processing such as the poly-A tail added to the end of naturally-occurring mRNAs. In some cases, an experimental design may require addition of a localization signal to provide for transport of the gene product to a particular subcellular location. Each of these elements is a fragment of a larger DNA molecule that is cut from a donor genome, or, in some cases, synthesized in a laboratory. Each piece is assembled with the others in a precise order and 5'-3' orientation into a cloning vector plasmid.

The promoter of any gene may be isolated as a DNA fragment and placed within a synthetic molecule, such as a plasmid, to direct the expression of a desired gene, assuming that the necessary conditions for stimulation of the promoter of interest can be provided. For example, the promoter sequences of the insulin gene may be isolated, placed in a cloning vector plasmid along with a reporter gene, and used to study the conditions required for expression of the insulin gene in an appropriate cell type. Alternatively, the insulin gene promoter may be joined with the protein coding-sequence of any gene of interest in a cloning vector plasmid, and used to drive expression of the gene of interest in insulin-expressing cells, assuming that all necessary elements are present within the DNA transgene so constructed.

A reporter gene is a particularly useful component of some types of transgenes. A reporter gene comprises nucleotide sequences encoding a protein that will be expressed under the direction of a particular promoter of interest to which it is linked in a transgene, providing a measurable biochemical response of the promoter activity. A reporter gene is typically easy to detect or measure against the background of endogenous cellular proteins. Commonly used reporter genes include but are not limited to LacZ, green fluorescent protein, and luciferase, and other reporter genes, many of which are well-known to those skilled in the art.

Introns, which are non-coding regions within mammalian genes, are not found in bacterial genomes, but are required for proper formation of mRNA molecules in mammalian cells. Therefore, any DNA construct for use in mammalian systems must have at least one intron. Introns may be isolated from any mammalian gene and inserted into a DNA construct, along with the appropriate splicing signals that allow mammalian cells to excise the intron and splice the remaining mRNA ends together.

An mRNA stabilization element is a sequence of DNA that is recognized by binding proteins that protect some mRNAs from degradation. Inclusion of an mRNA stabilization element will frequently enhance the level of gene expression from that mRNA in some mammalian cell types, and so can be useful in some DNA constructs or transgenes. An mRNA stabilization element can be isolated from naturally occurring DNA or RNA, or synthetically produced for inclusion in a DNA construct.

A localization signal is a sequence of DNA that encodes a protein signal for subcellular routing of a protein of interest. For example, a nuclear localization signal will direct a protein to the nucleus; a plasma membrane localization signal will direct it to the plasma membrane, etc. Thus, a localization signal may be incorporated into a DNA construct to promote the translocation of its protein product to the desired subcellular location.

A tag sequence may be encoded in a DNA construct so that the protein product will have an unique region attached. This unique region serves as a protein tag that can distinguish it from its endogenous counterpart. Alternatively, it can serve as an identifier that may be detected by a wide variety of techniques well known in the art, including, but not limited to, RT-PCR, immunohistochemistry, or *in situ* hybridization.

With a complex transgene, or with one that includes particularly large regions of DNA, there is an increased likelihood that there will be multiple restriction sites in these pieces of DNA. Recall that the restriction sites encoding any one hexanucleotide site occur every 4096 bp (4⁶). If a promoter sequence is 3000 bp and a gene of interest of 1500 bp are to be assembled into a cloning vector of 3000 bp, it is statistically very likely that many sites of 6 or less nucleotides will not be useful, since any usable sites must occur in only two of the pieces. Furthermore, the sites must occur in the appropriate areas of the appropriate molecules that are to be assembled. In addition, most cloning projects will need to have additional DNA elements added, thereby increasing the complexity of the growing molecule and the likelihood of inopportune repetition of any particular site. Since any restriction enzyme will cut at all of its restriction sites in a DNA molecule, if a restriction enzyme site reoccurs, all the inopportune sites will be cut along with the desired sites, disrupting the integrity of the molecule. Thus, each cloning step must be carefully planned so as not to disrupt the growing molecule by cutting it with a restriction enzyme that has already been used to incorporate a preceding element. And finally, when a researcher wishes to introduce a completed transgene into a mammalian organism, the fully-assembled transgene construct frequently must be linearized at a unique restriction site at at least one end of the transgene, thus requiring yet another unique site found nowhere else in the construct. Since most DNA constructs are designed for a single purpose, little thought is given to any future modifications that might need to be made, further increasing the difficulty for future experimental changes.

Traditionally, transgene design and construction consumes significant amounts of time and energy for several reasons, including the following:

1. There is a wide variety of restriction and HE enzymes available that will generate an array of termini; however most of these are not compatible with each other. Many restriction enzymes, such as EcoR1, generate DNA fragments with protruding 5' cohesive termini or "tails"; others (e.g., Pstl) generate fragments with 3' protruding tails, whereas still others (e.g., Ball) cleave at the axis of symmetry to produce blunt-ended fragments. Some of these will be compatible with the termini formed by cleavage with other restriction and/or HE enzymes, but the majority of useful ones will not. The termini that can be generated with each DNA fragment isolation procedure must be carefully considered in designing a DNA construct.

2. DNA fragments needed for assembly of a DNA construct or transgene must first be isolated from their source genomes, placed into plasmid cloning vectors, and amplified to obtain useful quantities. The step can be performed using any number of commercially-available or individually altered cloning vectors. Each of the different commercially available cloning vector plasmids were, for the most part, developed independently, and thus contain different sequences and restriction sites for the DNA fragments of genes or genetic elements of interest. Genes must therefore be individually tailored to adapt to each of these vectors as needed for any given set of experiments. The same DNA fragments frequently will need to be altered further for subsequent experiments or cloning into other combinations for new DNA constructs or transgenes. Since each DNA construct or transgene is custom made for a particular application with no thought or knowledge of how it will be used next, it frequently must be "retro-fitted" for subsequent applications.

3. In addition, the DNA sequence of any given gene or genetic element varies and can contain internal restriction sites that make it incompatible with currently available vectors, thereby complicating manipulation. This is especially true when assembling several DNA fragments into a single DNA construct or transgene.

While restriction enzymes can be used for manipulating genetic material, it is known that a MCS or other components of a transgene can be created by de novo synthesis, recombineering, and/or PCR terminator overhang cloning. One such method of synthesis of the component elements of a transgene includes the method disclosed by Jarrell et al. in U.S. Pat. No. 6,358,712. While Jarrell discloses a method for "welding" elements of a transgene together, the prior art does not teach a method or means to "unweld" and re-assemble these elements, once they have been assembled.

Therefore, it would be advantageous to provide a means to assemble each component of a transgene with the others in a precise order and 5'-3' orientation into a cloning vector plasmid. There is also a need for a system that would allow the user to rapidly assemble a number of DNA fragments into one molecule, despite a redundancy of restriction sites found at the ends, and within each, of these DNA fragments. It would also be useful to provide a simple means for rapidly altering the ends of selected DNA fragments in order to add restriction sites to them. Inclusion of single or opposing pairs of HE restriction sites would enhance the likelihood of having unique sites for cloning. A system that would also allow easy substitutions or removal of one or more of the fragments would add a level of versatility not currently available to users. Therefore, a "modular" system, allowing one to insert or remove DNA fragments into or out of "cassette" regions flanked by fixed rare restriction sites within the cloning vector would be especially useful, and welcome to the field of recombinant DNA technology.

Accordingly, one aspect of the present invention is to provide a method of rapidly assembling a DNA construct or transgene by using a cloning vector plasmid. Another aspect is to incorporate DNA fragments, also known as both "inserts" or "modules," such as a Promoter, Expression, and a 3'Regulatory nucleotide sequence, into a cloning vector plasmid, in a sequential manner.

### BRIEF SUMMARY OF THE INVENTION

The present invention provides a structure and methods to create a modular cloning vectors for the synthesis of a genetic domain module, a PE3 transgene, and other complicated DNA constructs, by providing a backbone within the modular cloning vectors having dedicated docking points known as gene pivots therein.

The present invention relates to a domain module docking vector consisting of a DNA cloning vector, comprising a multiple cloning (MC) module for sub-cloning a genetic material of interest into the MC module, the MC module comprising: a) a first gene pivot (GP) comprising at least two non-variable of greater than six nucleotides restriction sites operable to define the 5' portion of the MC module; b) a nucleic acid sequence comprising a multiple cloning site (MCS) comprising a plurality of restriction sites selected from common restriction sites that are unique within the domain module docking vector, to provide cloning sites for the cloning of the genetic material of interest into the MC module; and c) a second gene pivot comprising at least two non-variable restriction sites of greater than six nucleotides operable to define the 3' portion of the MC module, wherein the first gene pivot and the second gene pivot independently comprise at least 3, and not more than 4 non-variable restriction sites of greater than six nucleotides.

Also disclosed is a domain module vector consisting of a DNA cloning vector, comprising a domain module that comprises: a) a first gene pivot comprising at least two non-variable rare restriction sites operable to define the 5' portion of the domain module; b) a genetic module of interest consisting of a nucleic acid sequence comprising a genetic material of interest; and c) a second gene pivot comprising at least two non-variable rare restriction sites operable to define the 3' portion of the domain module.

Also disclosed is a PE3 docking vector consisting of a DNA cloning vector, comprising a PE3 cloning module that comprises at least one cloning module, configured for cloning at least a first domain module into the PE3 cloning module, the PE3 cloning module comprising: a) a first gene pivot comprising at least two non-variable rare restriction sites that upon cloning is operable to define the 5' portion of the at least first domain module; b) a stuffer module consisting of a first nucleic acid sequence comprising stuffer, that upon cloning is replaced by the first domain module; and c) a second gene pivot comprising at least two non-variable rare restriction sites that upon cloning is operable to define the 3' portion of the at least first domain module.

The present invention also relates to a vector comprising: a) a first gene pivot comprising at least two non-variable restriction sites of greater than six nucleotides that upon cloning is operable to define the 5' portion of a Promoter module; b) a first stuffer module consisting of a first nucleic acid sequence comprising stuffer, that upon cloning is replaced by the Promoter module; c) a second gene pivot comprising at least two non-variable restriction sites of greater than six nucleotides that upon cloning is operable to define a shared junction between the 3' portion of the Promoter module and the 5' portion of an Expression module; d) a second stuffer module consisting of a second nucleic acid sequence comprising stuffer, that upon cloning is replaced by the Expression module; e) a third gene pivot comprising at least two non-variable restriction sites of greater than six nucleotides that upon cloning is operable to define a shared junction between the 3' portion of the Expression module and the 5' portion of a 3' Regulatory module; f) a third stuffer module consisting of a third nucleic acid sequence comprising stuffer, that upon cloning is replaced by the 3' Regulatory module; and g) a fourth gene pivot comprising at least two non-variable restriction sites of greater than six nucleotides that upon cloning is operable to define the 3' portion of the 3' Regulatory module, wherein the first gene pivot and the second gene pivot independently comprise at least 3, and not more than 4 non-variable restriction sites of greater than six nucleotides.

Also disclosed is a PE3 vector consisting of a DNA cloning vector, comprising a PE3 module that comprises a Promoter module, an Expression module and a 3' Regulatory module, the PE3 module comprising: a) a first gene pivot comprising at least two non-variable rare restriction sites operable to define the 5' portion of a Promoter module; b) the Promoter module; c) a second gene pivot comprising at least two non-variable rare restriction sites operable to define a shared junction between the 3' portion of the Promoter module and the 5' portion of an Expression module; d) the Expression module; e) a third gene pivot comprising at least two non-variable rare restriction sites operable to define a shared junction between the 3' portion of the Expression module and the 5' portion of the 3' Regulatory module; f) the 3' Regulatory module; and g) a fourth gene pivot comprising at least two non-variable rare restriction sites operable to define the 3' portion of the 3' Regulatory module.

The present invention also provides that either one of the PE3 docking vector, PE3 vector, and PE3 multiple cloning (MC) docking vector, can comprises a means for releasing the PE3 module, typically comprising the Promoter domain, Expression domain, and 3' Regulatory domain modules flanked by the gene pivots, from the PE3. vector for insertion into a multigenic docking vector. One means for releasing and inserting the PE3 cloning module comprises a pair of homing endonucleases (HE) disposed in the PE3 docking vector or PE3 MC docking vector, that flank the nucleic acid sequence that comprises the PE3 cloning module. After insertion or cloning of the Promoter domain, Expression domain, and 3' regulatory domains into the PE3 cloning module, the pair of HE provide one means of excising the PE3 module from the PE3 vector, for insertion into a compatible docking position within the multigenic vector, the compatible docking position typically being defined by the same pair of HE. While any homing endonucleases can be used in either the first position (at the 5' end of the excised PE3 module) or the second position (at the 3' end of the excised PE3 module), a preferred HE pair consists of I-Ceu I in the first position, and ISce-I reversed at the second position. In other embodiments of the PE3 docking vector or PE3 MC docking vector, a different excision and insertion means can be used instead of HE. Such different excision and insertion means can include a pair of one or more non-variable rare restrictions sites, including a distinct pair of rare restrictions sites from those in the gene pivots, or the planking gene pivots (GP 1 and GP4) themselves. An insertion means employs recombineering, discussed hereinafter.

Also disclosed is a method for constructing a PE3 modular vector, comprising the steps of: a) providing a PE3 cloning vector comprising a PE3 cloning module, the PE3 cloning module comprising in sequence: a first gene pivot comprising at least two non-variable rare restriction sites, at least a first stuffer module consisting of a nucleic acid sequence comprising stuffer, and a second gene pivot; b) providing at least a first domain module vector comprising in sequence: the first gene pivot, a genetic module of interest consisting of a nucleic acid sequence comprising a genetic material of interest; and the second gene pivot; c) providing a first cognate restriction enzyme for one of the rare restriction sites of the first gene pivot and a second cognate restriction enzyme for one of the rare restriction sites of the second gene pivot; d) excising and isolating the genetic module of interest from the first domain module vector using the first and second cognate restriction enzymes; e) excising the first stuffer module from the PE3 cloning module of the PE3 cloning vector using the first and second cognate restriction enzymes; and f) ligating the genetic module of interest into PE3 cloning module. The method also provides for inserting a second genetic module of interest into the PE3 using a third gene pivot and cognate restriction enzymes to excise and ligate the second genetic module of interest into the PE3 cloning module. Similarly, a third genetic module of interest can be inserted into the PE3 using a third gene pivot and cognate restriction enzymes to excise and ligate the second genetic module of interest into the PE3 cloning module. The method provides a sequential arrangement of genetic modules of interest into the PE3 cloning vector. Typically the first, second and third genetic modules of interest correspond to a Promoter module, Expression module, and a 3'Regulatory module, respectively.

Also disclosed is a method for making a transgene comprising the steps of providing a cloning vector plasmid including a backbone, the backbone having at least a first, a second, a third, and a fourth docking point, the docking points being arranged sequentially in a 5'-3' direction and each having at least one non-variable rare restriction site operable to be cleaved by a restriction enzyme, cleaving the first docking point with a first restriction enzyme that corresponds to one of the first docking point's at least one non-variable rare restriction site, leaving the cleaved first docking point with an exposed 3' end, cleaving the second docking point with a second restriction enzyme that corresponds to one of the second docking point's at least one non-variable rare restriction site, leaving the cleaved second docking point with an exposed 5' end, providing a first insert comprising a 5' end, a nucleotide sequence of interest, and a 3' end, wherein the 5' end of the first insert is compatible to the exposed 3' end of the cleaved first docking point and the 3' end of the first insert is compatible to the exposed 5' end of the cleaved second docking point, and placing the first insert and the cleaved cloning vector plasmid into an appropriate reaction mixture to cause ligation and self-orientation of the first insert within the backbone between the first docking point and the second docking point, wherein the backbone is reassembled.

The second docking point can thereafter be cleaved with the second restriction enzyme, leaving the cleaved second docking point-with an exposed 3' end, the third docking point can be cleaved with a third restriction enzyme that corresponds to one of the third docking point's at least one non-variable rare restriction site, leaving the cleaved third docking point with an exposed 5' end, followed by the steps of providing a second insert comprising a 5' end, a nucleotide sequence of interest, and a 3' end, wherein the 5' end of the second insert is compatible to the exposed 3' end of the cleaved second docking point and the 3' end of the second insert is compatible to the exposed 5' end of the cleaved third docking point, and placing the second insert and the cleaved cloning vector plasmid into an appropriate reaction mixture to cause ligation and self-orientation of the second insert within the backbone between the second docking point and the third docking point, wherein the backbone is reassembled.

Further, this method can include the steps of thereafter cleaving the backbone at the third docking point with the third restriction enzyme, leaving the cleaved third docking point with an exposed 3' end, cleaving the fourth docking point with a fourth restriction enzyme that corresponds to one of the fourth docking point's at least one non-variable rare restriction site, leaving the cleaved fourth docking point with an exposed 5' end, providing a third insert comprising a 5' end, a nucleotide sequence of interest, and a 3' end, wherein the 5' end of the third insert is compatible to the exposed 3' end of the cleaved third docking point and the 3' end of the third insert is compatible to the exposed 5' end of the cleaved fourth docking point, and placing the third insert and the cleaved cloning vector plasmid into an appropriate reaction mixture to cause ligation and self-orientation of the third insert within the backbone between the fourth docking point and the second docking point, wherein the backbone is reassembled.

Also disclosed is a method for making a modular cloning vector plasmid for the synthesis of a trans gene or other complicated DNA construct, the method comprising the steps of providing a cloning vector plasmid including a backbone, the backbone having at least a first and a second docking point, the docking points each having at least one non-variable rare restriction site operable to be cleaved by a restriction enzyme, cleaving the first docking point with a first restriction enzyme that corresponds to one of the first docking point's at least one non-variable rare restriction site, leaving the cleaved first docking point with an exposed 3' end and the cleaved backbone with an exposed 5' end, providing a first insert comprising a 5' end, a nucleotide sequence of interest, and a 3' end, wherein the 5' end of the first insert is compatible to the exposed 3' end of the cleaved first docking point and the 3' end of the first insert is operable, when combined with the exposed 5' end of the cleaved backbone, to form a third docking point having at least one non-variable rare restriction site operable to be cleaved by a third restriction enzyme, placing the first insert and the cleaved cloning vector plasmid into an appropriate reaction mixture to cause ligation and self-orientation of the first insert within the backbone between the first docking point and the third docking point and upstream from the second docking point, wherein the backbone is reassembled, thereafter cleaving the third docking point with the third restriction enzyme, leaving the cleaved third docking point with an exposed 3' end and the cleaved backbone with an exposed 5' end, providing a second insert comprising a 5' end, a nucleotide sequence of interest, and a 3' end, wherein the 5' end of the second insert is compatible to the exposed 3' end of the cleaved third docking point, and the 3' end of the second insert is operable, when combined with the exposed 5' end of the cleaved backbone, to form a fourth docking point comprising at least one non-variable rare restriction site operable to be cleaved by a fourth restriction enzyme, placing the second insert and the cleaved cloning vector plasmid into an appropriate reaction mixture to cause ligation and self-orientation of the second insert within the backbone between the third docking point and the fourth docking point and upstream from the second docking point, wherein the backbone is reassembled, thereafter cleaving the fourth docking point with the fourth restriction enzyme, leaving the cleaved fourth docking point with an exposed 3' end, cleaving the second docking point with a second restriction enzyme corresponding to one of the second docking point's at least one non-variable rare restriction site, leaving the cleaved second docking point with an exposed 5' end, providing a third insert comprising a 5' end, a nucleotide sequence of interest, arid a 3' end, wherein the 5' end of the third insert is compatible to the exposed 3' end of the cleaved fourth docking point and the 3' end of the third insert is compatible to the exposed 5' end of the cleaved second docking point, and placing the third insert and the cleaved cloning vector plasmid into an appropriate reaction mixture to cause ligation and self-orientation of the third insert within the backbone between the fourth docking point and the second docking point, wherein the backbone is reassembled.

A further understanding of the nature and advantages of the present invention will be more fully appreciated with respect to the following drawings and detailed description.

### BRIEF DESCRIPTION OF THE DRAWINGS

The accompanying drawings, which are incorporated in and constitute a part of this specification, illustrate embodiments of the invention and, together with a general description of the invention given above, and the detailed description given below, serve to explain the principles of the invention.

FIG. 1 is a schematic illustration of a domain module docking vector containing a multiple cloning site for cloning in a genetic module.

FIG. 2 is a schematic illustration of a PE3 docking vector containing a domain cloning module for cloning in a domain module.

FIG. 3 is a schematic illustration of an alternative embodiment of a PE3 multiple cloning docking vector containing a multiple cloning site for cloning in genetic module,

FIG. 4 is a schematic illustration of a multigenic vector containing a means for cloning in a PE3 vector.

FIG. 5 is a another schematic illustration of the module concept of the invention.

FIG. 6 is a Docking Plasmid map.

FIG. 7 is a linear restriction map illustrating an example of restriction enzyme sites that can be included in the Docking Plasmid MCS.

FIG. 8 is a Multigenic (or Primary) Docking Plasmid map.

FIG. 9 is a linear restriction map illustrating an example of restriction enzyme sites that can be included in the Multigenic Docking Plasmid MCS.

FIG. 10 is a Shuttle Vector P ("SVP") plasmid map

FIG. 11 is a linear restriction site map illustrating an example of restriction enzyme sites that can be included in the Shuttle Vector P MCS.

FIG. 12 is a Shuttle Vector E ("SVE") plasmid map.

FIG. 13 is a linear restriction site map illustrating an example of restriction enzyme sites that can be included in the Shuttle Vector E MCS.

FIG. 14 is a Shuttle Vector 3' ("SV3") map.

FIG. 15 is a linear restriction site map illustrating an example of restriction enzyme sites that can be included in the Shuttle Vector 3' MCS.

FIG. 16 is a schematic illustration of a multigenic docking vector made and used with PE3 modules in accordance with the invention.

FIG. 17 is a schematic illustration of the insertion of the PE3 module into a multigenic docking vector employing gene pivots to provide a multigenic vector with the PE3 module.

FIG. 18 is a schematic illustration of the insertion of the PE3 module into a multigenic docking vector employing a multiple cloning site in the multigenic vector.

FIG. 19 is a schematic illustration of the insertion of the PE3 module into a multigenic docking vector employing a BstX-1 restriction sites in the multigenic vector.

FIG. 20 is a schematic illustration of the insertion of the PE3 module into a multigenic docking vector employing homing endonucleases in the multigenic vector.

FIG. 21 is a schematic illustration of the insertion of the PE3 module into a multigenic docking vector employing recombineering between the PE3 vector and the multigenic vector.

### BRIEF DESCRIPTION OF SEQUENCE LISTING

The accompanying sequence listings, which are incorporated in and constitute a part of this specification, illustrate embodiments of the invention and, together with a general description of the invention given above, and the detailed description given below, serve to explain the principles of the invention.

SEQ:ID 01 is an example of a nucleotide sequence for a PE3 Docking Plasmid MCS.

SEQ:ID 02 is an example of a nucleotide sequence for a PE3 Docking Plasmid.

SEQ:ID 03 is an example of a nucleotide sequence for a Primary Docking Plasmid MCS.

SEQ:ID 04 is an example of a nucleotide sequence for a Primary Docking Plasmid.

SEQ:ID 05 is an example of a nucleotide sequence for a Shuttle Vector P Plasmid MCS.

SEQ:ID 06 is an example of a nucleotide sequence for a Shuttle Vector P Plasmid.

SEQ:ID 07 is an example of a nucleotide sequence for a Shuttle Vector E Plasmid MCS.

SEQ:ID 08 is an example of a nucleotide sequence for a Shuttle Vector E Plasmid.

SEQ:ID 09 is an example of a nucleotide sequence for a Shuttle Vector 3 Plasmid MCS.

SEQ:ID 10 is an example of a nucleotide sequence for a Shuttle Vector 3 Plasmid.

### DEFINITIONS OF TERMS USED TO DESCRIBE THE INVENTION

For purposes of this invention, the following terms are defined as follows:

"Chromatin modification domain" (CMD) refers to nucleotide sequences that interact with a variety of proteins associated with maintaining and/or altering chromatin structure.

"Cloning" refers to the process of ligating a DNA molecule into a plasmid and transferring it an appropriate host cell for duplication during propagation of the host.

The term "cloning vector" refers to a circular DNA molecule minimally containing an Origin of Replication, a means for positive selection of host cells harboring the vector such as an antibiotic-resistance gene; and a multiple cloning site. A cloning vector can consist of à plasmid, a cosmid, an artificial chromosome (BAC, PAC, YAC, and others) or a viral vector backbone

"Cognate sequence" or "cognate sequences" refer to the minimal string of nucleotides required for a restriction enzyme to bind and cleave a DNA molecule or gene.

"Common" refers to any restriction site that occurs relatively frequently within a genome.

"Compatible to" refers a terminus or end, either 5' or 3', of a strand of DNA which can form hydrogen bonds with any other complementary termini either cleaved with the same restriction enzyme or created by some other method. Since any DNA that contains a specific restriction site for a restriction enzyme will be cut in the same manner as any other DNA containing the same sequence, those cleaved ends will be complementary and thus compatible. Therefore, the ends of any DNA molecules cut with the same restriction enzyme "match" each other in the way adjacent pieces of a jigsaw puzzle "match", and can be enzymatically linked together. Compatible ends will form a restriction site for a particular restriction enzyme when combined together.

"De novo synthesis" refers to the process of synthesizing double-stranded DNA molecules of any length by linking complementary single-stranded DNA molecules compatible overhangs that represent subsequences of the total desired DNA molecule.

"DNA construct" refers to a DNA molecule synthesized by consecutive cloning steps within a cloning vector plasmid, and is commonly used to direct gene expression in any appropriate cell host such as cultured cells *in vitro,* or a transgenic mouse *in vivo.* A transgene used to make such a mouse can also be referred to as a DNA construct, especially during the period of time when the transgene is being designed and synthesized.

"DNA fragment" refers to any isolated molecule of DNA, including but not limited to a protein-coding sequence, reporter gene, promoter, enhancer, intron, exon, poly-A tail, multiple cloning site, nuclear localization signal, or mRNA stabilization signal, or any other naturally occurring or synthetic DNA molecule. Alternatively, a DNA fragment may be completely of synthetic origin, produced *in vitro.* Furthermore, a DNA fragment may comprise any combination of isolated naturally occurring and/or synthetic fragments.

"Docking Plasmid" refers to a specialized cloning vector plasmid used in the invention to assemble DNA fragments into a DNA construct.

"Endonuclease" or "endonuclease enzyme", also commonly referred to herein as a "restriction enzyme," refers to a member or members of a classification of catalytic molecules that bind a restriction site encoded in a DNA molecule and cleave the DNA molecule at a precise location within or near the sequence.

The terms "endonuclease restriction site" or "restriction site" (as well as "cognate sequence" or "cognate sequences", above) refer to the minimal string of nucleotides required for a restriction enzyme to bind and cleave a DNA molecule or gene.

"Enhancer region" refers to a nucleotide sequence that is not required for expression of a target gene, but will increase the level of gene expression under appropriate conditions.

"Gene expression host selector gene" (GEH-S) refers to a genetic element that can confer to a host organism a trait that can be selected, tracked, or detected by optical sensors, PCR amplification, biochemical assays, or by cell/organism survival assays (resistance or toxicity to cells or organisms when treated with an appropriate antibiotic or chemical).

The terms "gene promoter" or "promoter" (P) can be used interchangeably and refer to a nucleotide sequence required for expression of a gene.

The terms "insert" and "module" are essentially interchangeable, with the only fine distinction being that an "insert" is inserted into the vector, and once it is inserted it is then more commonly called a "module". A module can then be removed from the vector. Also, the term insert is commonly used for an isolated module used as an insert into a modular acceptor vector.

"Intron" refers to the nucleotide sequences of a non-protein-coding region of a gene found between two protein-coding regions or exons.

"Localization signal" (LOC) refers to nucleotide sequences encoding a signal for subcellular routing of a protein of interest.

"Multiple cloning site" (MCS) refers to nucleotide sequences comprising at least one unique restriction site, and, more typically, a grouping of unique restriction sites, for the purpose of cloning DNA fragments into a cloning vector plasmid

The term "mRNA stabilization element" refers a sequence of DNA that is recognized by binding proteins thought to protect some mRNAs from degradation.

The term "operable to define" when referring to a group of restriction site means that when any one of the restriction sites in the group is cut with one of the cognate restriction enzymes, the overhang residue represents the 5' portion for the nucleotide sequences downstream from the group of restriction sites.

The term "Origin of Replication" (ORI) refers to nucleotide sequences that direct replication or duplication of a plasmid within a host cell

"PCR terminator over-hang cloning technology" refers to the process of amplifying genetic modules using the polymerase chain reaction in conjunction with single-stranded DNA primers with protected 5'over-hanging nucleotides that can serve as junction sites with complementary DNA over-hangs.

"Poly-A tail" refers to a sequence of adenine (A) nucleotides commonly found at the end of messenger RNA (mRNA) molecules. A Poly-A tail signal is incorporated into the 3' ends of DNA constructs or transgenes to facilitate expression of the gene of interest.

"Primer site" refers to nucleotide sequences that serve as DNA templates onto which single-stranded DNA oligonucleotides can anneal for the purpose of initiating DNA sequencing, PCR amplification, and/or RNA transcription.

The term "pUC19" refers to a plasmid cloning vector well-known to those skilled in the art, and can be found in the NCBI Genbank database as Accession # L09137.

"Random nucleotide sequences" refers to any combination of nucleotide sequences that do not duplicate sequences encoding other elements specified as components of the same molecule. The number of nucleotides required in the random sequences is dependent upon the requirements of the restriction enzymes that flank the random sequences. Most endonucleases require a minimum of 2 -4additional random sequences to stabilize DNA binding. It is preferred that the number of random sequences would be a multiple of 3, corresponding to the number of nucleotides that make up a codon. The preferred minimum number of random sequences is therefore 6, however, fewer or more nucleotides may be used.

"Rare" refers to a restriction site that occurs relatively infrequently within a genome.

"Recombination arm" refers to nucleotide sequences that facilitate the homologous recombination between transgenic DNA and genomic DNA. Successful recombination requires the presence of a left recombination arm (LRA) and a right recombination arm (RRA) flanking a region of transgenic DNA to be incorporated into a host genome via homologous recombination.

"Recombineering" refers to the process of using random or site-selective recombinase enzymes in conjunction with DNA sequences that can be acted on by recombinase enzymes to translocate a portion of genetic material from one DNA molecule to a different DNA molecule.

"Reporter gene" refers to a nucleotide sequences encoding a protein useful for monitoring the activity of a particular promoter of interest.

"Restriction endonuclease" or "restriction enzyme" refers to a member or members of a classification of catalytic molecules that bind a cognate sequence of DNA and cleave the DNA molecule at a precise location within that sequence.

"Shuttle Vector" refers to a specialized cloning vector plasmid used in the invention to make an intermediate molecule that will modify the ends of a DNA fragment.

"Tag sequence" (TAG) refers to nucleotide sequences encoding a unique protein region that allows it to be detected, or in some cases, distinguished from any endogenous counterpart.

"Untranslated region" (UTR) refers to nucleotide sequences encompassing the non-protein-coding region of an mRNA molecule. These untranslated regions can reside at the 5' end (5' UTR) or the 3' end (3' UTR) an mRNA molecule.

"Unique" refers to any restriction endonuclease or HE site that is not found elsewhere within a particular DNA molecule.

### DETAILED DESCRIPTION OF THE INVENTION

The present invention provides structures for and methods to create a multigenic docking vector, typically a plasmid vector and also referred to as a modular cloning vector for the synthesis of a PE3 transgene or other complicated DNA construct by providing a backbone having modular cloning or docking points therein. The invention is useful for assembling a variety of DNA fragments into a de novo DNA construct or transgene by using cloning vectors optimized to reduce the amount of manipulation frequently needed. The PE3 transgenic vector, herein referred to herein as a PE3 docking vector or Docking Plasmid, typically contains at least one multiple cloning site (MCS) and multiple sets of rare restriction and/or unique homing endonuclease ("HE") sites, arranged in a linear pattern. This arrangement defines a modular architecture that allows the user to place domain modules as inserts into a PE3 transgene construct without disturbing the integrity of DNA elements already incorporated into the Docking Plasmid in previous cloning steps.

While the present invention discloses and exemplifies modular vectors and methods for building genetic domain modules into PE3 transgene modules, and PE3 transgene modules for insertion into multigenic vectors, that are plasmid cloning vectors, similar methods can be used to build genetic domain modules and PE3 transgene modules in larger extrachromosomal DNA molecules such as cosmids or artificial chromosomes, including bacterial artificial chromosomes (BAC). The wide variety of genetic elements that can be incorporated into the plasmid cloning vectors also allow transfer of the final PE3 transgene products into a wide variety of host organisms with little or no further manipulation.

The present invention provides that both the 5' and 3' ends of each of the docking points (gene pivots) and each of the domain modules or inserts are all compatible with a corresponding end of another docking point or insert. For example, if a first docking point contains a restriction site for a non-variable rare restriction enzyme such as SgrAI and that docking point is thereafter cleaved, then a first insert intended to be inserted at the 3' end of the cleaved first docking point will contain a compatible 5' end to create a restriction site for SgrAI when the insert is combined with the first docking point. A second docking point within the plasmid may, for example, have a restriction site for a non-variable restriction enzyme such as SwaI. Any second insert will have at its 3' end a compatible nucleotide sequence to combine with the cleaved 5' end of the cleaved second docking point to create a restriction site for SwaI. Further, the 3' end of the first insert and the 5' end of the second insert, in order to be accommodatingly inserted into the modular cloning vector plasmid and also thereafter be removed at the same point, must contain compatible ends to create a third restriction site for a third non-variable rare restriction enzyme, such as, for example, PacI or SalI.

The domain module docking vectors of the invention, herein also known as "shuttle vectors" to shuttle in domain modules into the PE3 docking vector, contain a multiple cloning site with common restriction sites, which is flanked by at least rare restriction, and optionally with HE sites. The shuttle vectors are designed for cloning fragments of DNA into the common restriction sites between the rare restriction sites. The cloned fragments can subsequently be released by cleavage at the rare restriction (or HE) site or sites, and incorporated into the Pe3 docking vector using the same rare restriction and/or HE site or sites found in the shuttle vectors.

Thus, unlike conventional cloning vectors, the design of the MCS allows domain modules ("cassettes" or modules of DNA fragments) to be inserted into the modular regions of the PE3 docking vector (Docking Plasmid) using defined gene pivots comprising a group of rare restriction sites. Likewise, each can be easily removed using the same rare restriction and/or HE enzymes, and replaced with any other DNA fragment of interest. This feature allows the user to change the direction of an experimental project quickly and easily without having to rebuild the entire DNA construct. Therefore, the domain module and PE3 cloning vectors of the present invention allow the user to clone a DNA fragment into an intermediate domain module vector using common restriction sites, creating a cassette-accepting module, and to then transfer that modular fragment to the desired modular spot in the final construct by means of rare restriction sites. Furthermore, it allows future alterations to the molecule to replace individual modules in the PE3 Docking Plasmid with other cassette modules. The following descriptions highlight distinctions of the present invention compared with the prior art.

Each docking point (defined by a gene pivot) represents an area in which there is preferably at least two fixed non-variable rare restriction site, and more preferably fixed groupings of at least three non-variable rare restriction sites, and most preferably fixed groupings of not more than 4 non-variable rare restriction sites. A particular restriction site of each docking point is cleaved by its cognate restriction enzyme. This will create either a desired 5' or 3' end which is compatible with the complementary 5' or 3' end of one of the pre-constructed inserts containing a nucleotide sequence of choice, such as a Promoter, Expression or Regulator nucleotide sequence. At least two inserts, each of which have 5' and 3' ends that are compatible with the cleaved docking point of interest, can be added along with the cleaved cloning vector plasmid to an appropriate reaction mixture, and, assuming the proper thermodynamic milieu, the inserts can simultaneously, i.e. in a single step, become integrated into the cloning vector plasmid. During this singular addition and ligation reaction, the docking points are reformed and the cloning vector plasmid becomes modular, in that the docking points and the connection between the two modules can be re-cleaved with the appropriate restriction enzymes. The module can then later be removed, and a new module can be put in its place.

While it is possible to employ a single restriction site at the pivot point flanking the modules, there is a distinct possibility that a single "rare" restriction site is quite frequent within that particular DNA molecule. Recall that the frequency of restriction enzyme sites is a function of 4", as explained above. For example, a particular Promoter module in a cloning vector plasmid may contain a rare restriction site within its DNA sequence that also is present at its pivot point, therefore making it advantageous to have more than one restriction site at the pivot points. Thus, it is preferable to use more than one rare restriction site at a single pivot point, because the statistical probability of more than one of the restriction sites existing within the DNA sequence of the module of interest is much less. However, it is also preferable that no more than three or four restriction sites be located at a single pivot point, or else the "bunching together" of restriction sites will begin to affect the transcription/translation of the target molecule. With this number, the combination of restriction sites bunched together will be at most 18-24 bp long.

### Domain Module Vectors:

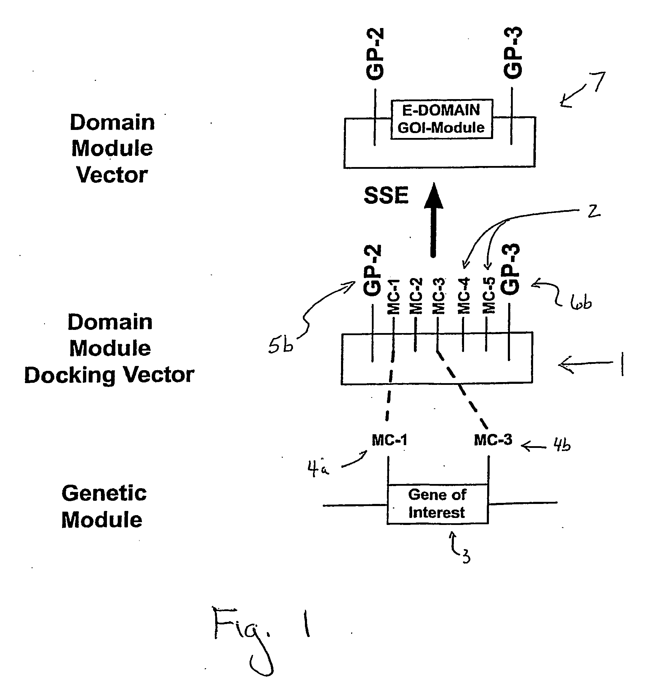

FIG. 1 shows a simplified representation of a first embodiment of the present invention, of a domain module docking vector 1. The vector 1 consists of a string of DNA having multiple cloning site 2, and is typically a plasmid. The domain module docking vector comprises a multiple cloning module (MC module) consisting of five cloning sites arranged in sequence, MC-1, MC-2, MC-3, MC-4, and MC-5. The multiple cloning site comprises a plurality of restriction sites that are independently selected from common restriction sites, as described herein after. Two of the restriction sites define a docking position for the genetic material of interest, illustrated as a gene of interest 3. The MC module enables the sub-cloning of a genetic material of interest between two of the restriction sites in the multiple cloning site of the MC module. The gene of interest 3 is typically released from a gene of interest vector (not shown), and includes a pair of cloning sites 4a and 4b, shown as MC-1 and MC-3.

The domain module docking vector also comprises a pair of restriction sites, referred to as gene pivots 5 and 6, that flank the multiple cloning site 2. The gene pivots each comprise at least two non-variable rare restriction sites, as defined herein after. The gene pivots 5 and 6 operate to define the 5' and 3' portions, respectively, of the MC module.

In a second embodiment of the invention, the cognate restriction enzymes MC-1 and MC-3 (not shown) can cut the gene of interest from its vector, and open the MC module at both the MC-1 and MC-3 MC sites, thereby allowing ligation of the gene of interest into between the MC-1 and MC-3 MC sites, thereby forming a domain module vector 7. In the illustrated embodiment, the gene of interest comprises an Expression domain, so that the domain module is an Expression module and the domain module vector is more particularly an Expression module vector. The Expression module vector comprises an Expression module 8, which comprises the first and second gene pivots 5 and 6 that flank a nucleic acid sequence comprising the sub-cloned gene of interest 3 that includes the Expression domain.

In the illustrated embodiment, the gene of interest comprises an Expression domain, wherein the first gene pivot (or 5' portion of the Expression domain) is hereinafter referred to as GP2 and the second gene pivot (or 3' portion of the Expression domain) as GP3. It can be understood that in alternative embodiments, the gene of interest can comprise a Promoter domain or a 3' Regulatory domain, whereby their sub-cloning provides a Promoter module within a Promoter module vector and a 3' regulatory module module within a 3' regulatory module vector, respectively. For Promoter module docking vectors, the first gene pivot (or 5' portion of the Promoter domain) is hereinafter referred to as GP 1 and the second gene pivot (or 3' portion of the Promoter domain) as GP2. For 3' Regulatory module docking vectors, the first gene pivot (or 5' portion of the 3' Regulatory domain) is hereinafter referred to as GP3 and the second gene pivot (or 3' portion of the 3' Regulatory domain) as GP4.

The gene pivots for any of the domain module docking vectors, including gene pivots Gyp1, GP2, GP3 and GP 4, can comprise rare restriction sites selected from the group consisting of AsiS I, Pac I, Sbf I, Fse I, Asc I, Mlu I, SnaB I, Not I, Sal I, Swa I, Rsr n, BSiW I, Sfo I, Sgr AI, AflIII, Pvu I, Ngo MIV, Ase I, Flp I, Pme I, Sda I, Sgf I, Srf I, and Sse8781 I, and more typically selected from the group consisting of AsiS I, Pac I, Sbf I, Fse I, Asc I, Mlu I, SnaB I, Not I, Sal I, Swa I, Rsr II, BSiW I. More typical embodiments of the gene pivot contain a series of at least 3, and no more than 4, rare restriction sites, and typically any one gene pivot does not include a rare restriction site of any other gene pivot.

In a preferred embodiment, gene pivot GP1 is selected from the group consisting of at least AsiS I, Pac I, and SbfI, gene pivot GP2 is selected from the group consisting of at least Fse I, Asc I, and Mlu I, gene pivot GP3 is selected from the group consisting of at least SnaB I, Not I, and Sal I, and gene pivot GP4 is selected from the group consisting of at least Swa I, Rsr II, and BSiW I. In a particular preferred embodiment, gene pivot GP1 consists of, in order, AsiS I, Pac I, and Sbf I; gene pivot GP2 consists of, in order, Fse I, Asc I, and Mlu I; gene pivot GP3 consists of, in order, SnaB I, Not I, and Sal I; and gene pivot GP4 consists of, in order, Swa I, Rsr II, and BSiW I.

It can also be understood that the multiple cloning site can comprise other combinations of restriction sites, in larger or small numbers of sites, in reverse order (for example, MC-5, MC-4, MC-3, MC-2, and MC-1), and using other types of cloning sites. The multiple closing sites can be varied to accommodate the pair of cloning sites for a particular gene of interest.

Also contemplated in an alternative embodiment is a library of domain module docking vectors, which can be used to readily subclone a wide variety of genes of interest The library of domain module docking vectors can be configured to serve as a dedicated Promotor domain module vector, Expression domain module vector, or 3' Regulatory module vector, depending on the type of gene pivots selected for the particular domain module docking vector. Each sublibrary of Promoter, Expression and 3' Regulatory domain module vectors have their own dedicated pair of gene pivots (namely, GP1 and GP2, GP2 and GP3, and GP3 and GP4) to ensure proper cloning of the respective domain modules into higher-order PE3 docking vectors are described hereinafter.

### PE3 Docking and PE3 MC Docking Vectors:

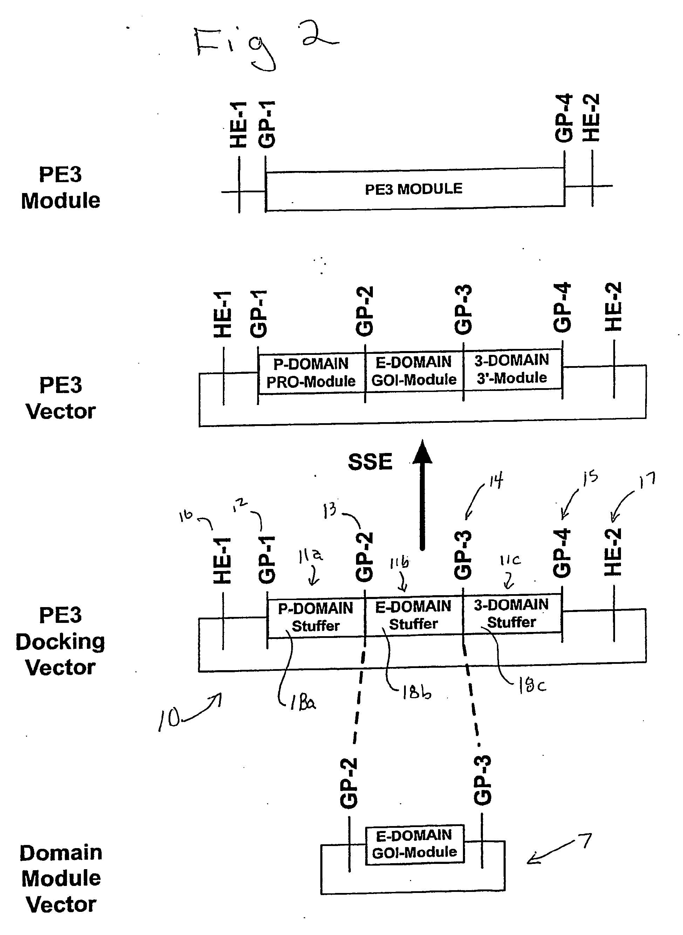

FIG. 2 shows a simplified representation of a third embodiment of the present invention, of a first PE3 docking vector 10. The PE3 docking vector 10 consists of a string of DNA having at least a first cloning module 11b, and is typically a plasmid. The at least first cloning module comprises at least a first and a second gene pivot, illustrated as GP2 and GP3, which flank a nucleic acid sequence that comprises stuffer 18b. A DNA stuffer domain is a random nucleotide sequence that does not encode for a restriction site or any other biological function resident within the PE3 docking vector. Stuffer DNA serves to increase the efficiency of restriction enzyme cutting activity by providing longer stretches of DNA to which the restriction enzyme can bind. This is important because many restriction enzymes cannot bind to and cut their cognate recognition sites if DNA lengths are limiting. The first and second gene pivots are as described herein before. The first cloning module 11 b is configured for cloning at least a first domain module into the PE3 cloning module. In the illustrated embodiment where the first cloning module is an Expression domain cloning module, the first and second gene pivots are represented as GP2 and GP3, as defined herein before. The first cloning module 11b comprising the stuffer, also termed the Expression stuffer module, provides a docking position for the insertion of an Expression domain module comprising compatible gene pivots. A compatible gene pivot is one which has identical restriction sites, or which comprises at least one unique rare restriction site in common with the gene pivot of the cloning module.

Also illustrated, a second cloning module 11 a is a Promoter stuffer module, having its first gene pivot 12 as GP1, and its second gene pivot 13 as GP2 that is a shared junction with the 5' portion of the cloning module 11b. The Promoter stuffer module 11a comprises stuffer and provides a docking position for the insertion of a Promoter domain module having compatible gene pivots, which replaces the Promoter stuffer module upon cloning. The third cloning module 11c is a 3' Regulatory stuffer module, having its first gene pivot 14 as GP3 that is a shared junction with the 3' portion of the cloning module 11b, and its second gene pivot 15 as GP4. The 3' Regulatory stuffer module 11c comprises stuffer and provides a docking position for the insertion of a 3' Regulatory domain module having compatible gene pivots, which replaces the 3' Regulatory stuffer module upon cloning.

In a fourth embodiment of the invention, the cognate restriction enzymes for one of the rare restriction sites contained in each gene pivot for one of the domain module vectors, illustrated the Expression module 7 can cut the rare restriction site from its residual vector, and likewise open the PE3 docking vector at the same rare restriction sites in the corresponding gene pivots GP2 and GP3, thereby replacing the Expression stuffer module with the Expression module. In a separate cloning event, suitable cognate restriction enzymes for the gene pivots for the Promoter module (not shown) and the corresponding gene pivots 12 and 13 of the Promoter stuffer module 11a can be used to ligate the Promoter module into the PE3 module, thereby replacing the Promoter stuffer module with the Promoter module. Likewise, the 3' Regulatory module can be ligated into the PE3 module.

In the present invention at least two, and more typically at least three rare restriction sites are used as docking pivots or gene pivot. The rarity of a rare restriction site in genetic sequences makes it unlikely that both rare restriction sites in a two-site gene pivot, and very unlikely that all three rare restriction sites in a three-site gene pivot, would be found in the docking vector. In the event that the DNA cloning vector is found to include one of the rare restrictions sites of the gene pivot, the skilled user can cut the gene pivot with one of the other cognate rare restriction enzymes. As an illustrate, if the GP2 gene pivot consists of Fse I, Asc I and MIu I, and the PE3 Docking vector or the E domain itself has Asc I within its sequence, then the skilled user can use either Fse I or MIu I to cut the gene pivots.

The DNA cloning vector of the PE3 vector also typically comprises a means for releasing the PE3 cloning module from the PE3 docking vector, for cloning into a multigenic docking vector, as will be described herein after.

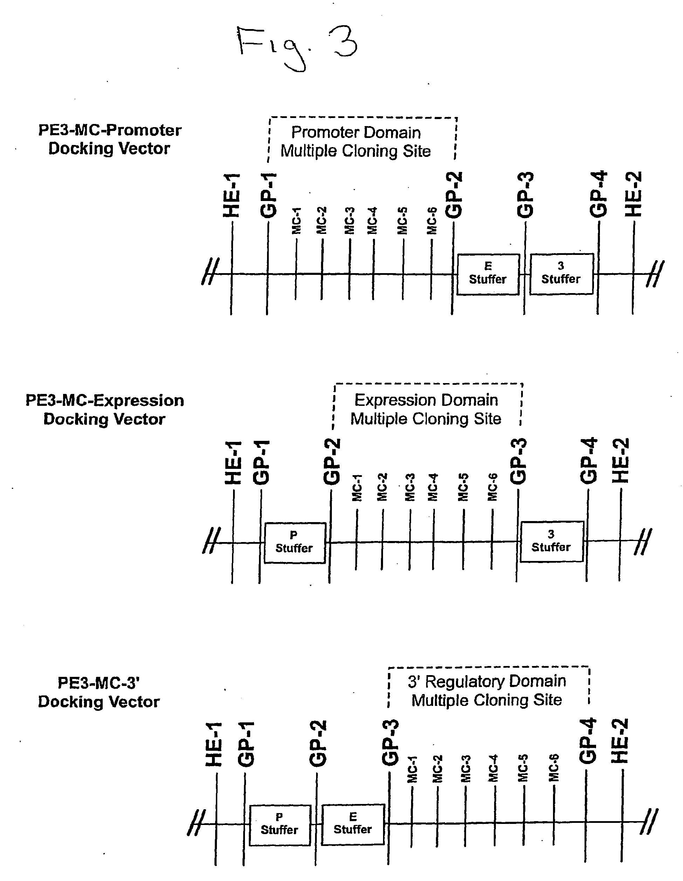

FIG. 3 shows an alternative embodiment for cloning in domain modules into a PE3 multiple cloning (MC) docking vector 20. A first PE3 MC docking vector, referred to as a PE3 MC-Promoter docking vector 20a, consists of a string of DNA having at least a first cloning module 21a, and is typically a plasmid. The first cloning module 21a comprises at least a first and a second gene pivot, illustrated as GP1 and GP2, which flank a nucleic acid sequence that comprises a multiple cloning site (MCS) comprising a plurality of restriction sites independently selected from common restriction sites, as described herein after. In the illustrated embodiment, the PE3 MC-Promoter docking vector 20 has three cloning modules, 21a, 21b and 21c, each bound by gene pivots GP1, GP2, GP3 and GP4, as described herein. The second and third cloning modules are shown as stuffer modules that contain a nucleic acid sequence that comprise stuffer.

The multiple cloning site consists of five cloning sites arranged in sequence, MC-1, MC-2, MC-3, MC-4, MC-5 and MC-6. Any two of the restriction sites can define a docking position for a genetic material of interest. In the PE3 MC docking vector 20a, the genetic material of interest is typically a Promoter domain. The selection of two cloning sites of interest can be based on the understanding that these restriction sites are unique within the PE3 MC docking vector 20a.

Alternative embodiments include a PE3 MC-Expression docking vector wherein the second cloning module 21 b comprises the multiple cloning site, with the remaining first and third cloning modules 21a and 21c comprising stuffer, and a PE3 MC-3 Regulatory docking vector wherein the third cloning module 21c comprises the multiple cloning site, with the remaining first and second cloning modules 21a and 21b comprising stuffer. In other alternative embodiments, two of the three cloning modules can comprise the multiple cloning site.

The sub-cloning of the multiple cloning site with a genetic material of interest, and the cloning of domain modules into the stuffer modules, in the PE3 docking vectors can be accomplished as described herein before.

The GP1, GP2, GP3 and GP4 gene pivots are as described herein before relative to the position of the respective modules in the Promoter, Expression and 3' Regulatory domain modules.

The present invention also provides for making PE3 vectors from modular domain vectors and a PE3 modular cloning vector. A first method for constructing a PE3 modular vector comprises the steps of:
a) providing a PE3 cloning vector comprising a PE3 cloning module, the PE3 cloning module comprising in sequence: a first gene pivot comprising at least two non-variable rare restriction sites, at least a first stuffer module consisting of a nucleic acid sequence comprising stuffer, and a second gene pivot;
b) providing at least a first domain module vector comprising in sequence: the first gene pivot, a genetic module of interest consisting of a nucleic acid sequence comprising a genetic material of interest; and the second gene pivot;
c) providing a first cognate restriction enzyme for one of the rare restriction sites of the first gene pivot and a second cognate restriction enzyme for one of the rare restriction sites of the second gene pivot;
d) excising and isolating the genetic module of interest from the first domain module vector using the first and second cognate restriction enzymes;
e) excising the first stuffer module from the PE3 cloning module of the PE3 cloning vector using the first and second cognate restriction enzymes; and
f) ligating the genetic module of interest into PE3 cloning module.

Further the method also provides, wherein the provided PE3 cloning module further comprises in sequence after the second gene pivot, a second stuffer module and a third gene pivot, with the further steps of:
g) providing a second domain module vector comprising in sequence the second gene pivot, a second genetic module of interest consisting of a nucleic acid sequence comprising a second genetic material of interest; and the third gene pivot;
h) providing a third cognate restriction enzyme for one of the rare restriction sites of the third gene pivot;
i) excising and isolating the second genetic module of interest from the second domain module vector using the second and third cognate restriction enzymes;
j) excising the second stuffer module from the PE3 cloning module of the PE3 cloning vector using the second and third cognate restriction enzymes; and
j) ligating the second genetic module of interest into PE3 cloning module.

Similarly, a third genetic module of interest can be inserted into the PE3 using a third gene pivot and cognate restriction enzymes to excise and ligate the second genetic module of interest into the PE3 cloning module. The method provides a sequential arrangement of genetic modules of interest into the PE3 cloning vector. Typically the first, second and third genetic modules of interest correspond to a Promoter module, Expression module, and a 3'Regulatory module, respectively. In a typical embodiment, the first gene pivot is GP1, the second gene pivot is GP2, the third gene pivot is GP3 and the fourth gene pivot is GP4, as described herein before.

In another embodiment, the method for making a PE3 cloning vector the method comprising the steps of:
a) providing a PE3 cloning vector having a backbone, the backbone comprising at least a first, a second, a third, and a fourth gene pivot, the gene pivots being arranged sequentially in a 5'-3' direction and each having at least two non-variable rare restriction site operable to be cleaved by a cognate restriction enzyme;
b) cleaving the first gene pivot with a first cognate restriction enzyme to one of the non-variable rare restriction site of the first gene pivot, leaving the cleaved first gene pivot with an exposed 3' end;
c) cleaving the second gene pivot with a second cognate restriction enzyme to one of the non-variable rare restriction site of the second gene pivot, leaving the cleaved second gene pivot with an exposed 5' end;
d) providing a first domain module comprising a 5' end, a first genetic material of interest, and first gene pivot a 3' end, wherein the 5' end of the first domain module is compatible to the exposed 3' end of the cleaved first gene pivot and the 3' end of the first domain is compatible to the exposed 5' end of the cleaved second gene pivot; and
e) placing the first domain module and the cleaved PE3 cloning vector into an appropriate reaction mixture to cause ligation and self-orientation of the first domain module within the backbone between the first gene pivot and the second gene pivot, wherein the backbone is reassembled.

The method can further comprising the steps of:
f) thereafter cleaving the second gene pivot with the second cognate restriction enzyme, leaving the cleaved second gene pivot with an exposed 3' end;
g) cleaving the third gene pivot with a third cognate restriction enzyme to one of the rare restriction sites of the third gene pivot, leaving the cleaved third gene pivot with an exposed 5' end;
h) providing a second domain module comprising a 5' end, a first genetic material of interest, and a 3' end, wherein the 5' end of the second domain module is compatible to the exposed 3' end of the cleaved second gene pivot and the 3' end of the second domain module is compatible to the exposed 5' end of the cleaved third gene pivot; and
i) placing the second domain module and the cleaved PE3 cloning vector into an appropriate reaction mixture to cause ligation and self-orientation of the second domain module within the backbone between the second gene pivot and the third gene pivot, wherein the backbone is reassembled.

The method even further can provide the steps of:
j) thereafter cleaving the backbone at the third gene pivot with the third cognate restriction enzyme, leaving the cleaved third gene pivot with an exposed 3' end;
k) cleaving the fourth gene pivot with a fourth cognate restriction enzyme to one of the rare restriction sites of the fourth gene pivot, leaving the cleaved fourth gene pivot, with an exposed 5' end;
l) providing a third domain module comprising a 5' end, a third genetic material of interest, and a 3' end, wherein the 5' end of the third domain module is compatible to the exposed 3' end of the cleaved third gene pivot, and the 3' end of the third domain module is compatible to the exposed 5' end of the cleaved fourth gene pivot; and
m) placing the third domain module and the cleaved PE3 cloning vector into an appropriate reaction mixture to cause ligation and self-orientation of the third domain module within the backbone between the third gene pivot, and the fourth gene pivot, wherein the backbone is reassembled.

Typically the first, second and third genetic modules of interest correspond to a Promoter module, Expression module, and a 3'Regulatory module, respectively. In a typical embodiment, the first gene pivot is GP1, the second gene pivot is GP2, the third gene pivot is GP3 and the fourth gene pivot is GP4, as described herein before.

### Multigenic Cloning Vectors:

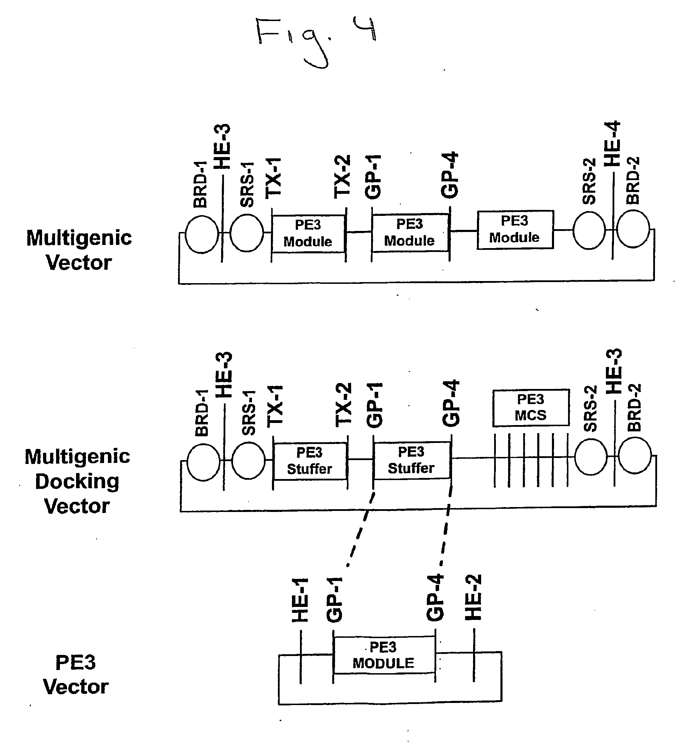

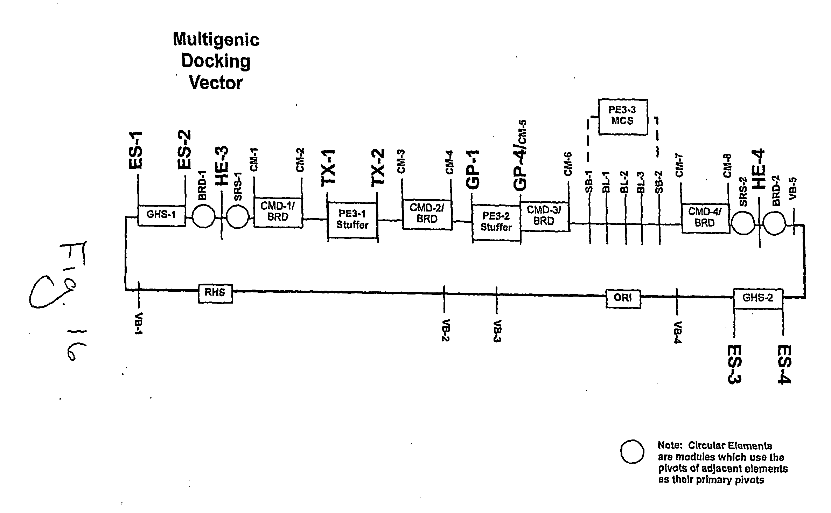

The PE3 docking and MC docking vectors of the present invention provide a means of easily and rapidly assembling one or a plurality of PE3 transgenes of interest. The invention also provides for a means of rapidly and easily inserting one or a plurality of the PE3 modules into a modular multigenic vector. FIG. 4 shows a simplified schematic of the insertion of a PE3 module into a multigenic docking vector that comprises one or (as illustrated) a plurality of PE3 stuffer modules that can be readily released to enable insertion of the one or more PE3 modules. Multigenic vectors are described in greater detail hereinafter and in FIGS. 16-21.

Restriction sites used in the invention are chosen according to a hierarchy of occurrence. In order to determine the frequency of restriction site occurrence, DNA sequence information corresponding to nineteen different genes was analyzed using Vector NTI software. This search covered a total of 110,530 nucleotides of DNA sequence. Results from these analyses were calculated according to the number of instances of a restriction site occurring within the analyzed 110,530 nucleotides. Restriction sites were then assigned a hierarchical designation according to four classifications, wherein "common" sites occur greater than 25 times per 110,530 nucleotides, "lower-frequency 6 bp sites" occur between 6-24 times per 110,530 nucleotides, and "rare" sites occur between 0-5 times per 110,530 nucleotides. A partial list of "suitable" enzymes is hereby listed according to their occurrence classifications:

### Common restriction sites:

Suitable common restriction enzymes can include, but are not limited to, Ase I, BamH I, Bgl II, Blp I, BstX I, EcoR I, Hinc II, Hind III, Nco I, Pst I, Sac I, Sac II, Sph I, Stu I, Xba I.

Suitable restriction sites that have a 6 bp recognition site, but have a lower frequency of occurrence, can include, but are not limited to, Aar I, Aat II, Afl II, Age I, ApaL I, Avr II, BseA I, BspD I, BspE I, BstB I, Cla I, Eag I, Eco0109 I, EcoR V, Hpa I, Kpn I, Mfe I, Nar I, Nde I, NgoM IV, Nhe I, Nsi I, Pml I, SexA I, Sma I, Spe I, Xho I.

### Rare Restriction sites:

Suitable rare enzymes can include, but are not limited to, Acl I, Nru I, Pac I, Pme I, Sbf I, Sfi I, PI-Sce I, I-Sce I, I-Ceu I, PI-Psp I, I-Tli I, Fse I, Sfo I , Asi SI, Sgr AI, Asc I, Mlu I, Sna BI, Not I, Sal I, Swa I, Rsr II, Bsi WI, AflIII, Pvu I, Ngo MIV, Ase I, Flp I, Pme I, Sda I, Sgf I, Srf I, and Sse8781 I.

### Homing Endonucleases:

Suitable Homing Endonucleases can include, but are not limited to, I-SceI, PI-Sce I, I-Ceu I, PI-Psp I, I-Chu I, I-Cmoe I, I-Cpa I, I-Cpa II, I-Cre I, I-Cvu I, I-Dmo I, I-LIa I, I-Mso I, I-Nan I, I-Nit I, I-Nja I, I-Pak I, I-Por I, I-Ppo I, I-Sca I, I-Sce II, I-Sce III, I-Sce IV, I-Sce V, I-Sce VI, I-Ssp68031, I-Tev I, I-Tev II, I-Two I, PI-Mga I, PI-Mtu I, PI-Pfu I, PI-Pfu II, PI-Pko I, PI-Pko II, PI-Tfu I, PI-Tfu II, PI-Thy I, and PI-Tli

### DETAILED EMBODIMENTS OF THE INVENTION

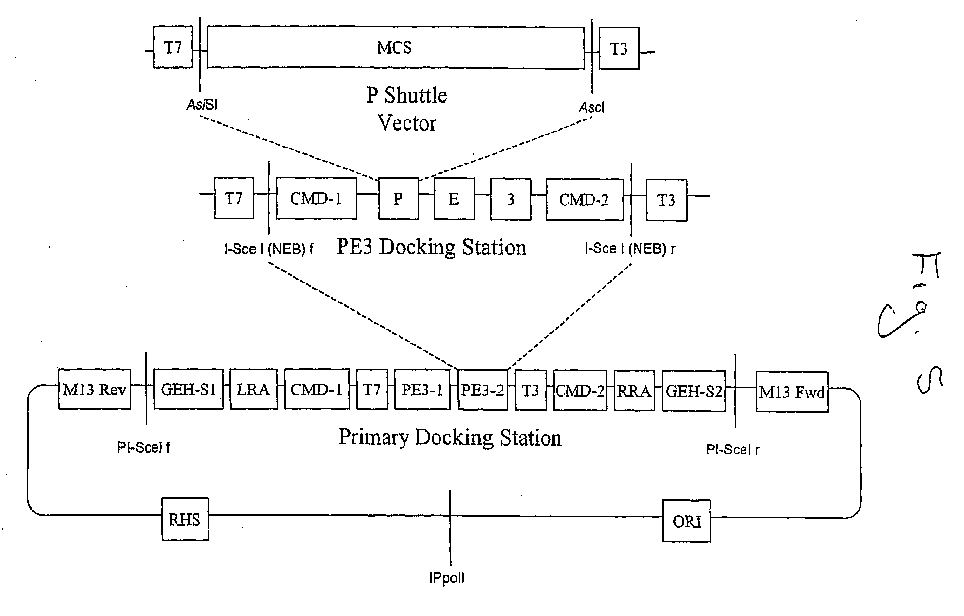

Individual components of a PE3 vector or transgene are the Promoter enhancer module (designated as "P"), the expressed protein module ("E"), and/or the 3'Regulatory region module ("3"), and can be assembled as modules transferred from domain module (or "shuttle") vectors into a PE3 docking vector (which can also be referred to as a docking station). FIG. 5 shows a Promoter module, within a Promoter vector, which can be excised and inserted into a PE3 docking vector are a predetermined docking position. As also shown in FIG. 5, if higher orders of complexity are needed, the assembled PE3 modular transgenes, or other nucleotide sequences, can then be transferred into a multigenic docking vector (also referred to as a Primary Docking Station Plasmid) or into a locus targeting docking vector. Each of the five types of cloning vector plasmids (Promoter module, Expression module, and 3' Regulatory module vectors, PE3 docking vectors and multigenic vectors) will be explained in greater detail to illustrate the components incorporated into each.

Domain module docking vectors, also known as "shuttle vectors," which contain a multiple cloning site typically comprising common restriction sites, and flanked by a gene pivot, typically comprising a rare restriction site, and/or HE sites. The domain module docking vector is constructed from a pUC19 backbone, and has the following modifications to the pUC19 backbone, wherein the sequences are numbered according to the pUC19 Genbank sequence file, Accession # L09137:
1. Only sequences from 806 to 2617 (Afl3-Aat2) are used in the Docking Plasmid,
2. The BspH1 site at 1729 in pUC 19 is mutated from TCATGA to GCATGA,
3. The Ac11 site at 1493 in pUC19 is mutated from AACGTT to AACGCT,
4. The Acl1 site at 1120 in pUC19 is mutated from AACGTT to CACGCT,
5. The Ahd1 site in pUC19 is mutated from GACNNNNNGTC to CACNNNNNGTC,

Sequences encoding BspH1/I-Ppo I/BspH1 are inserted at the only remaining BspH1 site in pUC19 following the mutation step 2 in the list above..

The three individual shuttle vectors of the present invention, Promoter Module Docking Vector, Expression Module Docking Vector, and 3' Regulatory Module Docking Vector, are described in particular embodiments, and identified as Shuttle Vector Promoter/intron ("SVP"), Shuttle Vector Expression ("SVE"), and Shuttle Vector 3'Regulatory ("SV3"), respectively. Each is described more fully below.

### Shuttle Vector P

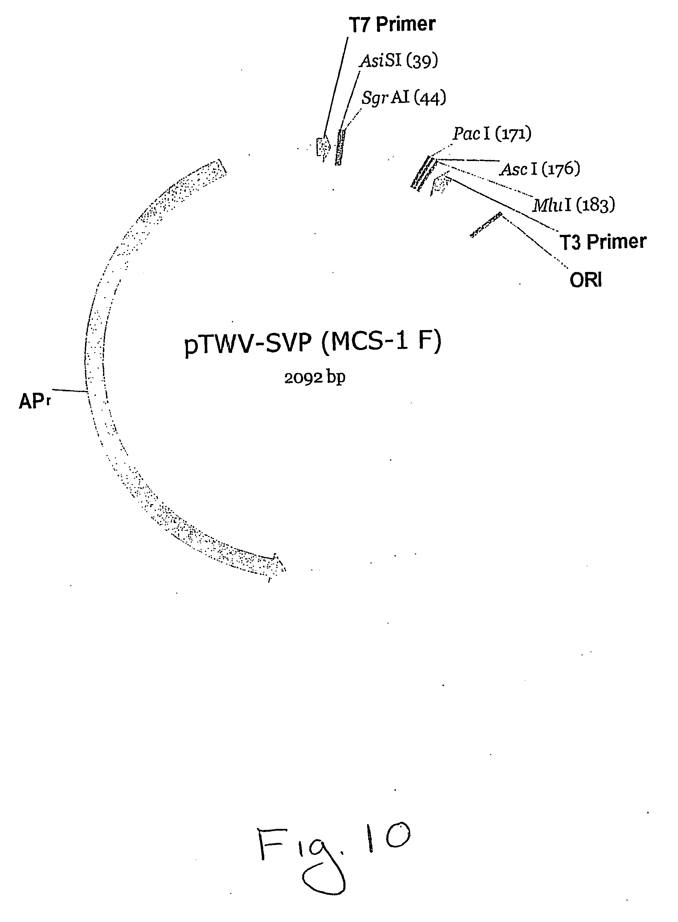

A SVP is shown in FIG. 10, and it is a cloning vector plasmid that can be used to prepare promoter and intron sequences for assembly into a PE3 docking vector (transgene construct) as described herein before.

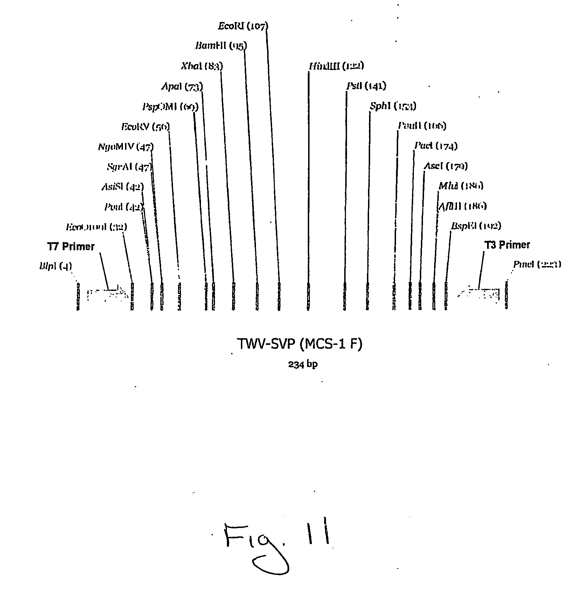

FIG. 11 shows an example of a SVP Plasmid comprising the following sequential elements in a MCS, in the order listed:
1. Two non-variable and unique, common restriction sites that define a 5' insertion site for the mutated pUC19 vector described above (for example, AatII and BlpI),
2. A T7 primer site,
3. A non-variable and unique, common restriction site that allows efficient cloning of a shuttle vector module downstream of the T7 primer site (for example, Eco0109I),
4. A fixed grouping of non-variable rare restriction sites that define the 5' portion of the promoter module (for example, AsiSI and SgrAI),
5. A variable MCS comprising any grouping of common or rare restriction sites that are unique to the shuttle vector (for example, the series of restriction sites illustrated in FIG. 11),
6. A fixed grouping of non-variable rare restriction sites that define the 3' portion of the promoter module (for example, PacI, AscI, and MluI)
7. A non-variable and unique, common restriction site that allows efficient cloning of a shuttle vector module upstream of the T3 primer site (for example, BspEI)
8. A reverse-orientation T3 primer site, and
9. Two non-variable and unique, common restriction sites that define a 3' insertion site for the mutated pUC19 vector described above (for example, PmeI and SapI).

In an alternative example, the fixed grouping of non-variable rare restriction sites that define the 5' portion of the promoter module (the GP1 gene pivot) comprise AsiS I, Pac I and Sbf I, and the rare restriction sites that define the 3' portion of the promotor module (the GP2 gene pivot) comprise Fse I, Asc I and Mlu I. In a more specific example, the GP1 gene pivot consists of the sequence of AsiS I, Pac I and Sbf I, and the GP2 gene pivot consists of the sequence of Fse I, Asc I and Mlu I.

### Shuttle Vector E (SVE)

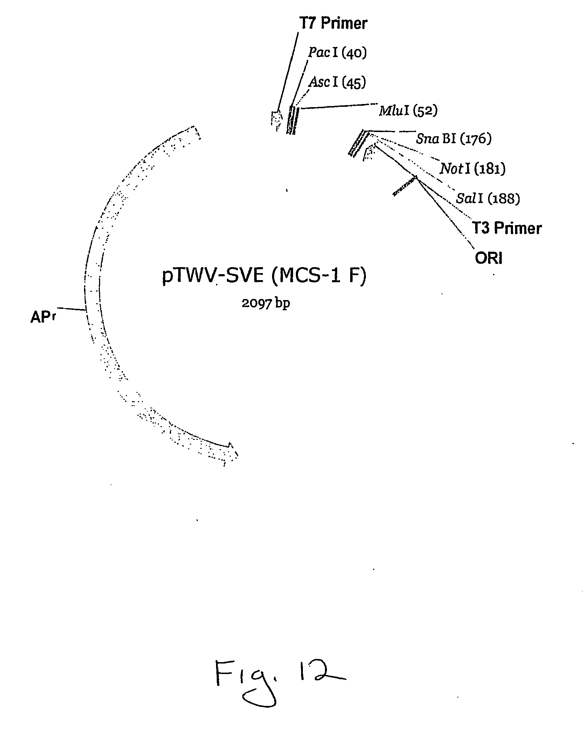

A SVE shown in FIG. 12, is a cloning vector plasmid that can be used to prepare sequences to be expressed by the transgene, for modular assembly into the PE3 vector (transgene construct) as described herein before.

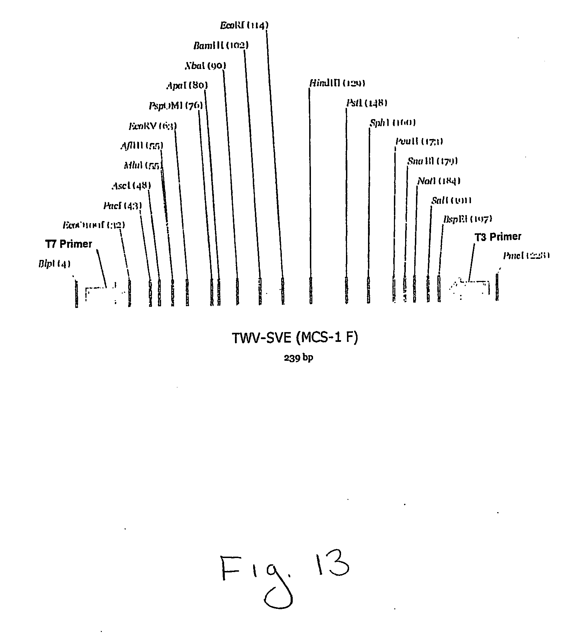

FIG. 13 shows an example of an SVE plasmid comprising the following sequential elements in the MCS, in the order listed:
1. Two non-variable and unique, common restriction sites that define a 5' insertion site for the mutated pUC19 vector described above (for example, BlpI),
2. A T7 primer site,
3. A non-variable and unique, common restriction site that allows efficient cloning of a shuttle vector module downstream of the T7 primer site (for example, Eco0109I),
4. A fixed grouping of non-variable rare restriction sites that define the 5' portion of the expression module (for example, PacI, AscI, and MluI),
5. A variable MCS consisting of any grouping of common or rare restriction sites that are unique to the shuttle vector (for example, the series of restriction sites illustrated in FIG. 13),
6. A fixed grouping of non-variable rare restriction sites that define the 3' portion of the expression module (for example, SnaBI, NotI, and SalI),
7. A non-variable and unique, common restriction site that allows efficient cloning of a shuttle vector module upstream of the T3 primer site (for example, BspEI)
8. A reverse-orientation T3 primer site, and
9. Two non-variable and unique, common restriction sites that define a 3' insertion site for the mutated pUC 19 vector described above (for example, PmeI).

In an alternative example, the fixed grouping of non-variable rare restriction sites that define the 5' portion of the expression module (the GP2 gene pivot) comprise Fse I, Asc I and Mlu I, and the rare restriction sites that define the 3' portion of the expression module (the GP3 gene pivot) comprise SnaB I, Not I, and Sal I. In a more specific example, the GP2 gene pivot consists of the sequence of Fse I, Asc I and Mlu I and the GP3 gene pivot consists of the sequence of SnaB I, Not I, and Sal I.

### Shuttle Vector 3 (SV3)

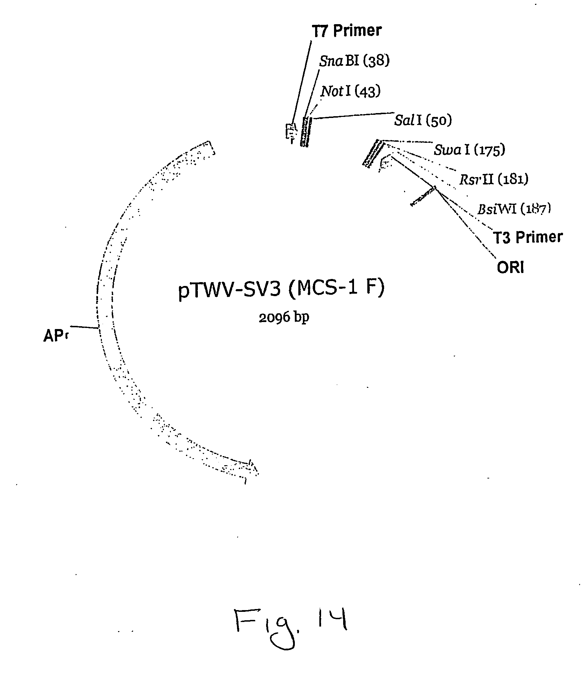

SV3, shown in FIG. 14, is a cloning vector plasmid that can be used to prepare 3'Regulatory sequences, for assembly into a PE3 vector (transgene construct) as described herein before.

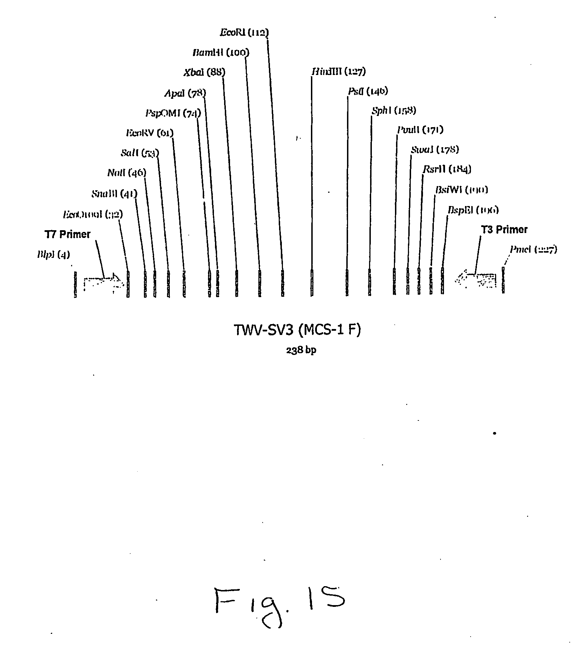

In FIG. 15, an example of an SV3 plasmid can comprise the following elements in the MCS, in the order listed:
1. Two non-variable and unique, common restriction sites that define a 5' insertion site for the mutated pUC19 vector described above (for example, BlpI),
2. A T7 primer site,
3. A non-variable and unique, common restriction site that allows efficient cloning of a shuttle vector module downstream of the T7 primer (for example, Eco0109I),
4. A fixed grouping of non-variable rare restriction sites that define the 5' portion of the 3'Regulatory module (for example, SnaBI, NotI, and SalI),
5. A variable MCS consisting of any grouping of common or rare restriction sites that are unique to the shuttle vector (for example, the series of restriction sites illustrated in FIG. 15),
6. A fixed grouping of non-variable rare restriction sites that define the 3' portion of the 3'Regulatory module (for example, SwaI, RsrII, and BsiWI),
7. A non-variable and unique, non-rare restriction site that allows efficient cloning of a shuttle vector module upstream of the T3 primer site (for example, BspEI),
8. A reverse-orientation T3 primer site, and
9. Two non-variable and unique, non-rare restriction sites that define a 3' insertion site for the mutated pUC19 vector described above (for example, PmeI).

In an alternative example, the fixed grouping of non-variable rare restriction sites that define the 5' portion of the expression module (the GP3 gene pivot) comprise SnaB I, Not I, and Sal I, and the rare restriction sites that define the 3' portion of the expression module (the GP4 gene pivot) comprise Swa I, Rsr II and BsiW I. In a more specific example, the GP3 gene pivot consists of the sequence of SnaB I, Not I, and Sal I and the GP4 gene pivot consists of the sequence of Swa I, Rsr II and BsiW I.

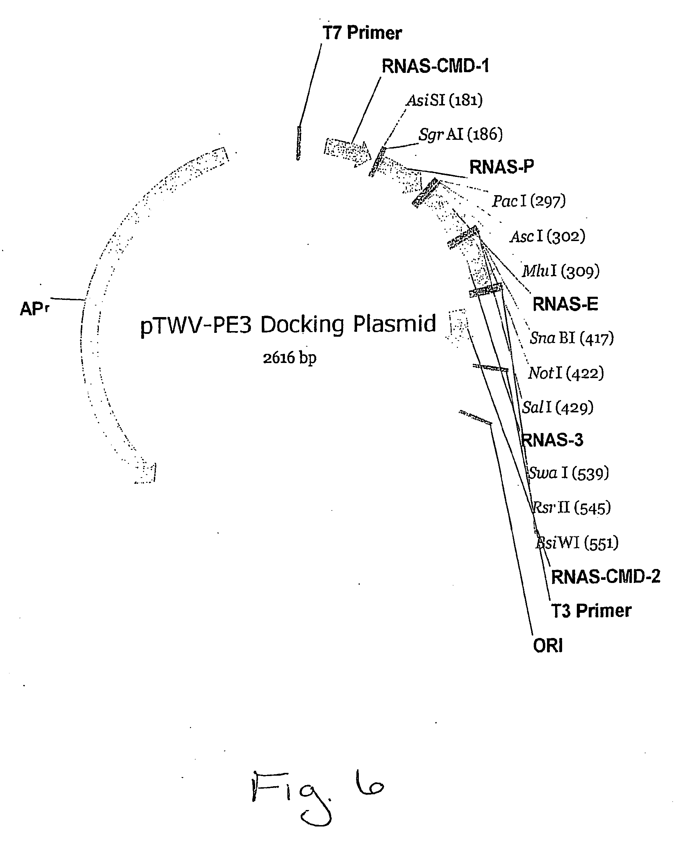

The PE3 docking vector (PE3 Docking Plasmid) shown in FIG. 6 comprises a pUC19 backbone modified as in the above example.

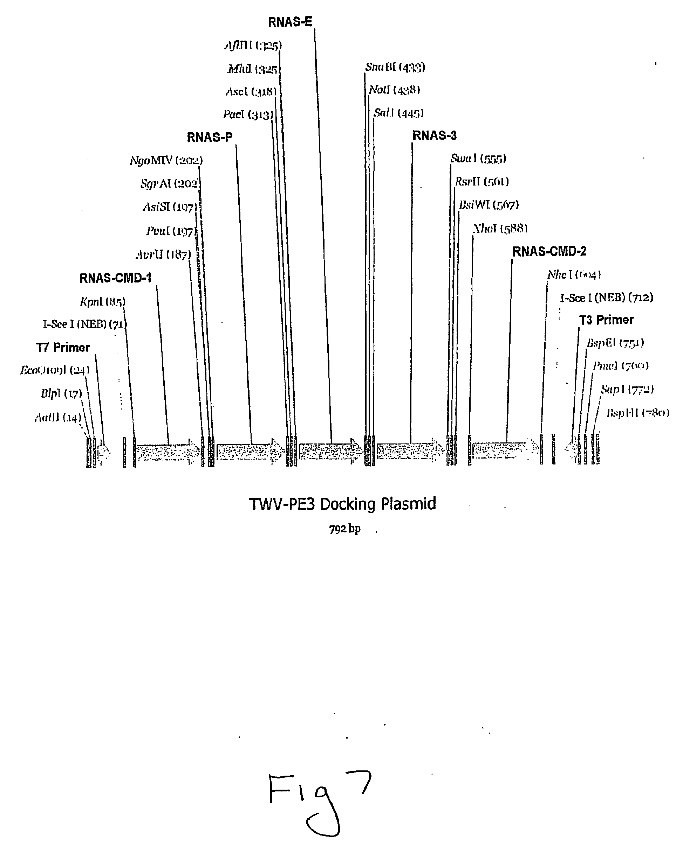

The multiple cloning site (MCS) in the PE3 Docking Plasmid, shown in FIG. 7, comprises the following sequential elements, in the order listed:
1. Three non-variable and unique common restriction sites that define a 5' insertion site for the mutated pUC19 vector described above (for example, Aat II, Blp I, and EcoO109 I),
2. A T7 primer site.
3. A first unique HE site (for example, I-SceI (here, in a forward orientation)),
4. A pair of non-variable and unique, common restriction sites flanking random nucleotide sequences that can serve as a chromatin modification domain acceptor module (RNAS-CMD-1) (for example, Kpn I and Avr II),
5. A fixed grouping of non-variable rare restriction sites that define the 5' portion of the promoter module. Examples of non-variable rare restriction sites for use as pivots at the 5' end of the promoter module preferably include AsiS I, Pacl, and Sfo I, but can also include PvuI, AsiSI, and SgrA I.
6. Random nucleotide sequences that can serve as a Promoter/intron acceptor module (RNAS-P),
7. A fixed grouping of non-variable rare restriction sites that define the shared junction between the 3' portion of the Promoter/intron module and the 5' portion of the Expression module. Examples of non-variable rare restriction sites for use as pivots at the 3' end of the promoter module preferably include Fsel, AscI and MluI, but can also include Pacl.
8. Random nucleotide sequences that can serve as an expression acceptor module (RNAS-E),
9. A fixed grouping of non-variable rare restriction sites that define the junction of the 3' portion of the Expression module and the 5' portion of the 3'Regulatory module (for example, SnaB I, Not I, and Sal I),
10. Random nucleotide sequences that can serve as a 3'Regulatory domain acceptor module (RNAS-3),
11. A fixed grouping of non-variable rare restriction sites that define the 3' portion of the 3'Regulatory module (for example, Swa I, Rsr II, and BsiW I),
12. A pair of non-variable and unique, common restriction sites flanking a random nucleotide sequence of DNA that can serve as a chromatin modification domain acceptor module (RNAS-CMD-2) (for example, Xho I and Nhe I),
13. A second unique HE site (here, identical to that in item 3 above (ISce1), and also in reverse orientation). It has recently been observed, however, that if the two unique HE sites are identical to one another and in reverse orientation, there can occur an unfavorable recombination event. Therefore, it may be preferred to use a second unique HE site that is not identical to the first unique HE site.
14. A T3 primer site in reverse orientation, and
15. Four non-variable and unique common restriction sites that define a 3' insertion site for the mutated pUC19 vector described above (for example, BspE I, Pme I, Sap I, and BspH I).

In an alternative example, the fixed grouping of non-variable rare restriction sites that define the 5' portion of the promoter module (the GP1 gene pivot) comprise AsiS I, Pac I and Sbf I; the rare restriction sites that define the 3' portion of the promoter module and the 5' portion of the expression module (the GP2 gene pivot) comprise Fse I, Asc I and Mlu I; the rare restriction sites that define the 3' portion of the expression module and the 5' portion of the 3' regulatory module (the GP3 gene pivot) comprise SnaB I, Not I, and Sal I; and the rare restriction sites that define the 3' portion of the 3' regulatory module (the GP4 gene pivot) comprise Swa I Rsr II and BsiW I.

In a more specific example, the GP1 gene pivot consists of the sequence of AsiS I, Pac I and Sbf I, the GP2 gene pivot consists of the sequence of Fse I, Asc I and Mlu I, the GP3 gene pivot consists of the sequence of SnaB I, Not I, and Sal I, and the GP4 gene pivot consists of the sequence of Swa I Rsr II and BsiW I.

### The Multigenic Docking Vector:

FIG. 16 shows a multigenic docking vector that comprises a pUC19 backbone identical to that of the PE3 Docking Vector except for the inclusion of additional restriction sites to create module boundaries to flank vector backbone modules. These backbone modules include a Replication Host Selector (RHS) gene module and a Origin of Replication gene module (ORI). The RHS is defined by the vector backbone pivot (VB) Aat II, a DNA sequence encoding a Replication Host Selector Gene, and two additional vector backbone pivots Age I and Avr II. The RHS module in FIG. 16 is defined by the vector backbone pivot VB-1 = Aat II, the RHS Module = Ampicillin Resistance gene, and the vector backbone pivots VB-2 = Age I and VB-3 = Avr II. The ORI is defined by the vector backbone pivots Age I and Avr II, a DNA sequence encoding an origin of replication, and the vector backbone pivot Pme I. The ORI module in FIG. 16 is defined by the vector backbone pivots VB-2 = Age I and VB-3 = Avr II, the ORI module = pUC 10 origin of replication, and the vector backbone pivots VB-4 = Pme I.

The multigenic docking vector is defined by the precise placement of random nucleotide sequences and non-random nucleotide sequences defining common, rare, and homing endonuclease sites for the purpose of creating a diverse array of domain acceptor regions. These module acceptors are defined by the following classes: GHS, BRD, SRS, CMD, (CMD/BRD), PE3 Stuffer, PE3 MCS. These modules are defined as:
- A.: GHS = Genome Expression Host Selector Gene (ex: NEO or PURO)
- B.: BRD = A non-random nucleic acid sequence that can be used as a site for homologous recombination in "recombineering-competent" bacterial cell lines
- C.: SRS = A non-random nucleic acid sequence that can be used as a site for site-specific homologous recombination mediated by a site-specific recombinase gene product (ex: Cre/Lox or Flp/Frt)
- D.: CMD = A non-random nucleic acid sequence that can alter the chromatin structure of a host genome (ex: HS4 chicken beta globin insulator)
- E.: CMD/BRD = A non-random nucleic acid sequence encoding a chromatin modification domain that can serve as a DNA sequence for homologous recombination in "recombineering-competent" bacterial cell lines
- F.: PE3 Stuffer = a random nucleotide sequence that does not encode for a restriction site or any other biological function resident within the multigenic docking vector
- G.: PE3 MCS = a non-random nucleotide sequence consisting of a plurality of restriction sites unique to the multigenic docking vector

These modules are flanked by restriction sites that can be used to make module docking vectors. These module-associated restriction sites can be defined according to the following classifications:
- A.: VB = restriction sites defining the boundaries of a vector backbone module
- B.: ES = restriction sites defining the boundaries of a genome expression host selector module
- C.: TX = synthetic BstX I sites generating overhangs compatible to specific rare and homing endonuclease sites (ex: I-Ceu I, I-Sce I, Sbf I)
- D.: CM = restriction sites defining the boundaries of a chromatin modification module
- E.: SB = restriction sites defining the boundaries of a site-specific recombination module
- F.: BL = restriction sites producing blunt ends

One embodiment of the invention can be defined by the nucleotide sequence residing between the vector backbone pivots VB-1 and VB-5, wherein VB-1 = Aat II and VB-5 = BspE I.
- 1.: VB-1 is Aat II
- 2.: No sequence or Random nucleotide sequence
- 3.: ES-1 = SexA I
- 4.: Random nucleotide sequence stuffer (or GHS Module)
- 5.: ES-2 = Srf I
- 6.: Random nucleotide sequence stuffer (or BRD Module)
- 7.: HE-3 = 1-Ppo I
- 8.: Random nucleotide sequence stuffer (or SRS Module)
- 9.: No sequence or Random nucleotide sequence stuffer
- 10.: CM-1= Kpn I
- 11.: Random nucleotide sequence (or CMD Module)
- 12.: CM-2 = Sac I
- 13.: No sequence or Random nucleotide sequence stuffer
- 14.: TX-1 = Bst X I (I-Ceu I) forward orientation
- 15.: Random nucleotide sequence stuffer (or PE3 Module)
- 16.: TX-2 = Bst X I (I-Sce I) reverse orientation
- 17.: No sequence or Random nucleotide sequence stuffer
- 18.: CM-3 = Mfe I
- 19.: Random nucleotide sequence stuffer (or CMD Module)
- 20.: CM-4 = Sac II
- 21.: No sequence or Random nucleotide sequence stuffer
- 22.: GP-1 = (AsiS 1/Pac 1/Sbf I)
- 23.: Random nucleotide sequence stuffer (or PE3 Module)
- 24.: G-4/CM-5 = (Swa I/Rsr II/BsiW I)
- 25.: Random nucleotide sequence stuffer (or CMD Module)
- 26.: CM-6 = Nar I, which has same motif as Kas I
- 27.: No sequence or Random nucleotide sequence stuffer
- 28.: SB-1 = Nsi I, the sticky overhang is compatible to Sbf I
- 29.: No sequence or Random nucleotide sequence stuffer
- 30.: BL-1 = Sfo I
- 31.: No sequence or Random nucleotide sequence stuffer
- 32.: BL-2 = Pvu II
- 33.: No sequence or Random nucleotide sequence stuffer
- 34.: BL-3 = Nru I
- 25.: No sequence or Random nucleotide sequence stuffer
- 36.: SB-2 = BsrG I, the sticky overhang is compatible to BsiW I
- 37.: No sequence or Random nucleotide sequence stuffer
- 38.: CM-7 = SpeI
- 39.: Random nucleotide sequence stuffer (or CMD Module)
- 40.: CM-8 = Sph I
- 41.: Random nucleotide sequence stuffer (or SRS Module)
- 42.: HE-4 = PI-Sce I (forward orientation)
- 43.: Random nucleotide sequence stuffer (or BRD Module)
- 44.: VB-5 = BspE I

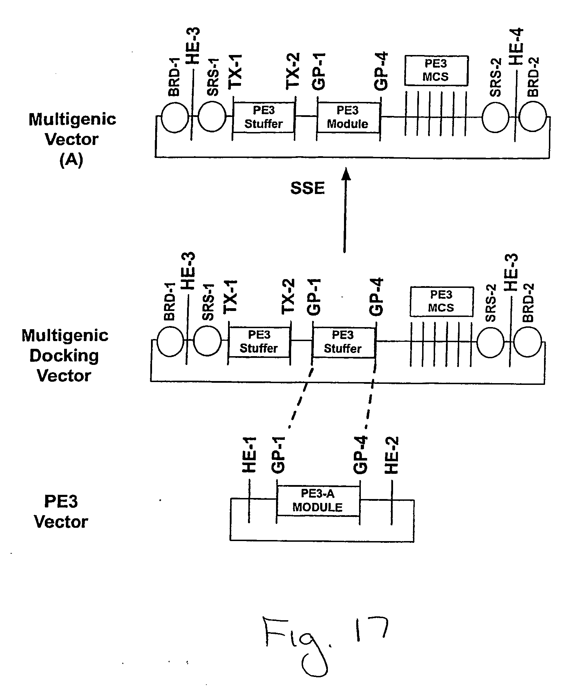

FIGS. 17, 18, and 19 illustrate how the multigenic docking vector in FIG. 16 can serve as a DNA backbone, using conventional subcloning methodologies, to build a multigenic vector having two or more PE3 modules. This example of building a multgenic vector with three distinct PE3 modules employs the preferred order of subcloning events based upon the statistical probability that the pivots needed to create a PE3 acceptor domain in the docking vector will not be present in any PE3 module(s) already included within the multigenic vector backbone.

The preferred order of subcloning PE3 modules into the multigenic docking vector illustrated in FIG 16 is as follows:

1. A PE3 module should be subcloned into the PE3-2 stuffer domain first, and this PE3 module should be chosen according to the ordered absence of the following sites:
i. Nsi I or BsrG I
ii. Sfo I, Pvu II, or Nru I
iii. BstX I

2. The second PE3 module should be subcloned into the PE3-3 domain next, and this PE3 module should be chosen according to the ordered absence of the following sites:
iv. Sbf I or BsiW I
v. Sbf I alone
vi. BsiW I
vii. BstX I

3. The third PE3 module should be subcloned into the PE3-1 domain

A PE3 module that fulfills the criteria listed in item 1 can be subcloned into the multigenic docking vector by independently preparing the vector backbone and PE3 module by digesting with two restriction enzymes, wherein one enzyme recognizes a single restriction site in the GP-1 domain and the second enzyme recognizes a single restriction site in the GP-4 domain. The enzymes selected must not cut within the PE3 module. An example of this process is illustrated in FIG. 17. One method for subcloning the PE3-A module in FIG. 17 into the PE3-2 Stuffer domain requires the preparation of a linearized vector backbone produced by cutting the multigenic docking vector with the enzymes AsiS I and Rsr II. This molecular biology protocol produces DNA docking points with AsiS I-specific and Rsr II-specific sticky overhangs. A linear DNA fragment containing the desired PE3-A module can also be prepared by digesting with AsiS I and Rsr II. The resulting vector backbone and PE3-A module can be biochemically linked together using a DNA ligase enzyme to generate a multigenic vector containing the PE3-A module.

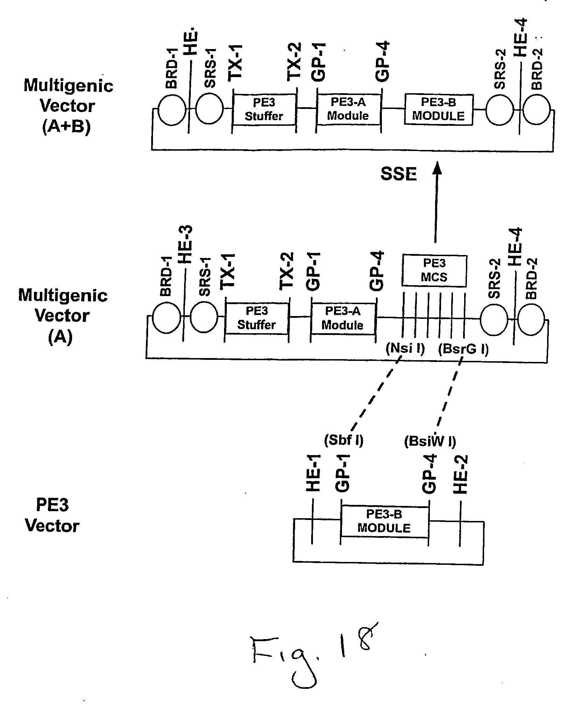

FIG. 18 introduces one methodology by which a PE3 module that fulfills the criteria in item 2 can be subcloned into the multigenic docking vector containing the PE3-A module at the PE3-3 Multiple Cloning Site (PE3-3 MCS). In this example, the PE3-A module does not contain either a Nsi I or a BsrG I site (designated in FIG. 16 as SB-1 and SB-2, respectively), and the PE3-B module does not contain a Sbf I or a BsiW I site. As depicted in FIG 18, a linearized PE3-B module is produced by digesting the PE3-B module vector with Sbf I and BsiW I. The linearized multigenic vector backbone is prepared by digesting with Nsi I and BsrG I. The complementary sticky cohesive ends in the PE3-B module and the multigenic vector backbone can be linked together biochemically using a DNA ligase enzyme. The resulting DNA molecule consists of the multigenic vector wherein the PE3-A and PE3-B modules are now a contiguous part of the DNA molecule.

FIG. 18 represents only one of numerous strategies for subcloning a PE3 module into the PE3-3 Stuffer domain in the multigenic docking vector. Other subcloning strategies include the production of linearized PE3 modules and linearized multigenic docking vectors displaying sticky/blunt, blunt/sticky, or blunt/blunt docking points. These alternative subcloning strategies are predicted to display lower success rates for generating ligation products than would sticky/sticky strategies. These alternative strategies can be chosen if the structure of either the PE3-A or PE3-B modules does not meet one or more of the criteria listed in item 2.

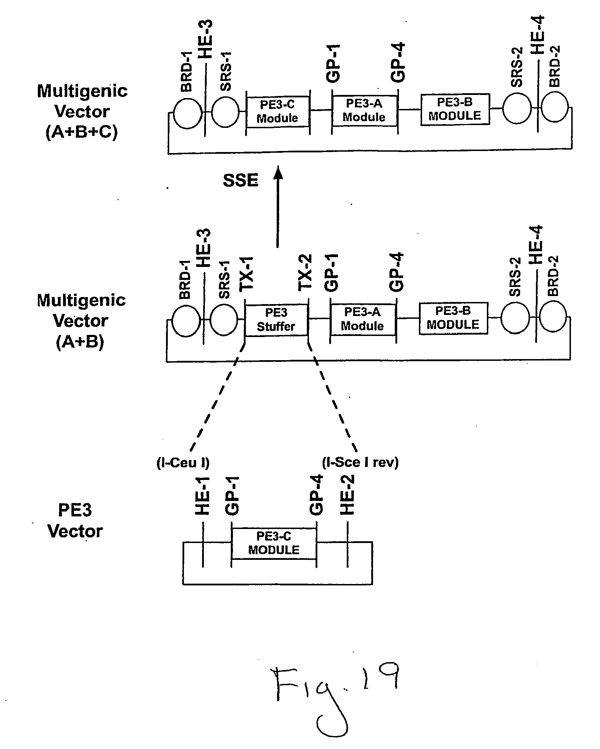

FIG. 19 illustrates how a PE3-C module can be subcloned into the multigenic vector containing the PE3-A and PE3-B modules. This strategy requires that neither the PE3-A nor the PE3-B contain a BstX I site. In this strategy, the multigenic docking vector is linearized by digesting with BstX I. The PE3-C module is linearized by digesting with the homing endonucleases I-Ceu I and I-Sce I. The resulting linear PE3-C module and multigenic docking vector are linked together biochemically using a DNA ligase enzyme. The resulting DNA molecule consists of the multigenic vector wherein the PE3-A, PE3-B, and PE3-C modules are now a contiguous part of the DNA molecule.

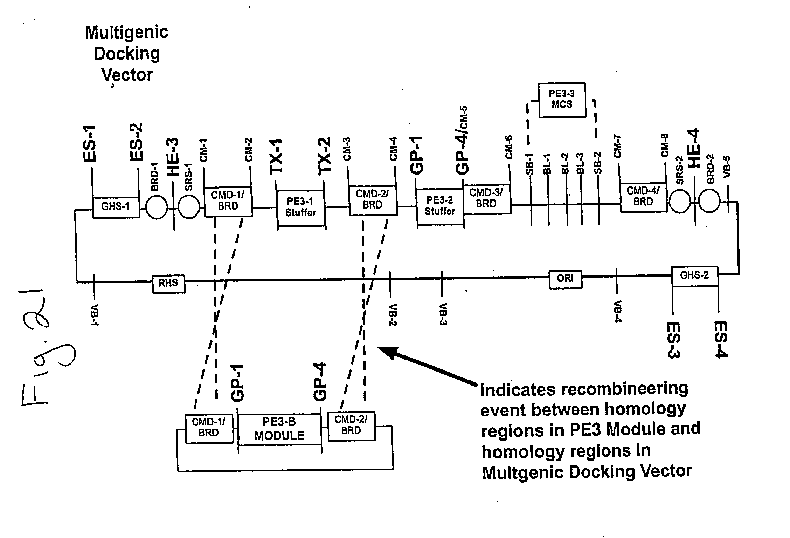

FIG 21 illustrates how a PE3 module flanked by either chromatin modification domains (CMD) or bacterial recombineering domains (BRD) can be introduced into a multigenic docking vector employing bacterial recombineering methodologies. In this example, the PE3 module replaces the PE3-1 Stuffer Domain through the action of homologous recombination between the CMD/BRD domains flanking both the PE3 module in the PE3 Module Vector and the PE3-1 Stuffer Domain in the Multigenic Docking Vector. This strategy requires the use of ancillary DNA vectors or special bacterial strains wherein the expression of recombination enzymes can be induced by environmental changes (chemical, temperature, or metabolic).

Another embodiment of the invention, illustrated in FIG. 16, can be defined by the nucleotide sequence residing between the vector backbone pivots VB-1 and VB-5, wherein VB-1= Aat II and VB-5 = BspE I.
- 1.: VB-1 is Aat II
- 2.: No sequence or Random nucleotide sequence
- 3.: ES-1= SexA I
- 4.: Random nucleotide sequence stuffer (or GHS Module)
- 5.: ES-2 = Srf I
- 6.: Random nucleotide sequence stuffer (or BRD Module)
- 7.: HE-3 = I-Ppo I
- 8.: Random nucleotide sequence stuffer (or SRS Module)
- 9.: No sequence or Random nucleotide sequence stuffer
- 10.: CM-1 = Kpn I
- 11.: Random nucleotide sequence (or CMD Module)
- 12.: CM-2 = Sac I
- 13.: No sequence or Random nucleotide sequence stuffer
- 14.: HE-1 = I-Ceu I forward orientation
- 15.: Random nucleotide sequence stuffer (or PE3 Module)
- 16.: HE-2 = 1-Sce I reverse orientation
- 17.: No sequence or Random nucleotide sequence stuffer
- 18.: CM-3 = Mfe I
- 19.: Random nucleotide sequence stuffer (or CMD Module)
- 20.: CM-4 = Sac II
- 21.: No sequence or Random nucleotide sequence stuffer
- 22.: GP-1= (AsiS I/Pac 1/Sbf I)
- 23.: Random nucleotide sequence stuffer (or PE3 Module)
- 24.: G-4/CM-5 = (Swa I/Rsr II/BsiW I)
- 25.: Random nucleotide sequence stuffer (or CMD Module)
- 26.: CM-6 = Nar I, which has same motif as Kas I
- 27.: No sequence or Random nucleotide sequence stuffer
- 28.: TX-1 = Bst X I (I-Ceu I) forward orientation
- 29.: SB-1 = Nsi I, the sticky overhang is compatible to Sbf I
- 30.: No sequence or Random nucleotide sequence stuffer
- 31.: BL-1 = Sfo I
- 32.: No sequence or Random nucleotide sequence stuffer
- 33.: BL-2 = Pvu II
- 34.: No sequence or Random nucleotide sequence stuffer
- 35.: BL-3 = Nru I
- 36.: No sequence or Random nucleotide sequence stuffer
- 37.: SB-2 = BsrG I, the sticky overhang is compatible to BsiW I
- 38.: No sequence or Random nucleotide sequence stuffer
- 39.: TX-2 = Bst X I (I-Sce I) reverse orientation
- 40.: No sequence or Random nucleotide sequence stuffer
- 41.: CM-7 = Spe I
- 42.: Random nucleotide sequence stuffer (or CMD Module)
- 43.: CM-8 = Sph I
- 44.: Random nucleotide sequence stuffer (or SRS Module)
- 45.: HE-4 = PI-Sce I (forward orientation)
- 46.: Random nucleotide sequence stuffer (or BRD Module)
- 47.: VB-5 = BspE I

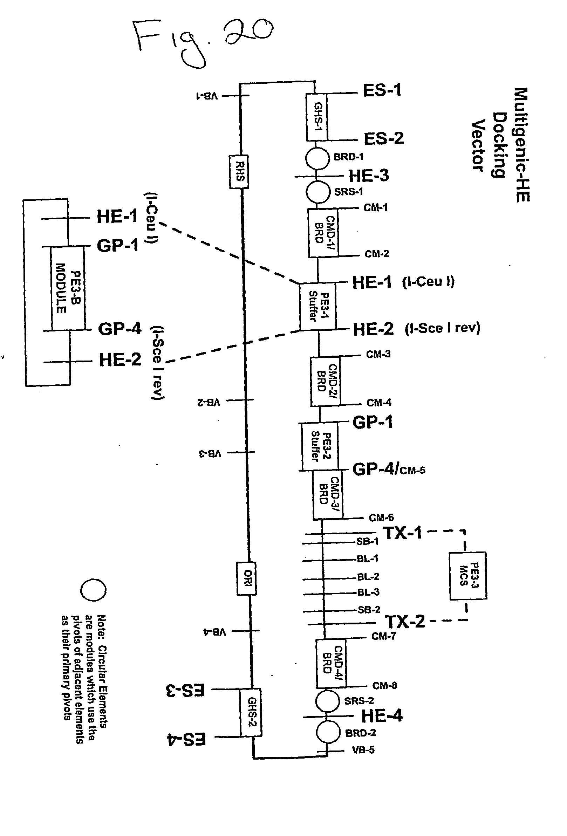

FIG. 20 illustrates how homing endonucleases can be used to introduce a PE3 module into the Multigenic Docking Vector described above. In this example, the PE3 module and the multigenic docking vector are linearized by digesting with the homing endonucleases I-Ceu I and I-Sce I. The resulting linear PE3 module can be linked to the linear multigenic docking vector biochemically using a DNA ligase enzyme. This subcloning strategy can be used if a PE3 module within a multigenic docking vector, or being introduced into a docking vector, does not meet one or more of the criteria listed in items 1 through 3.

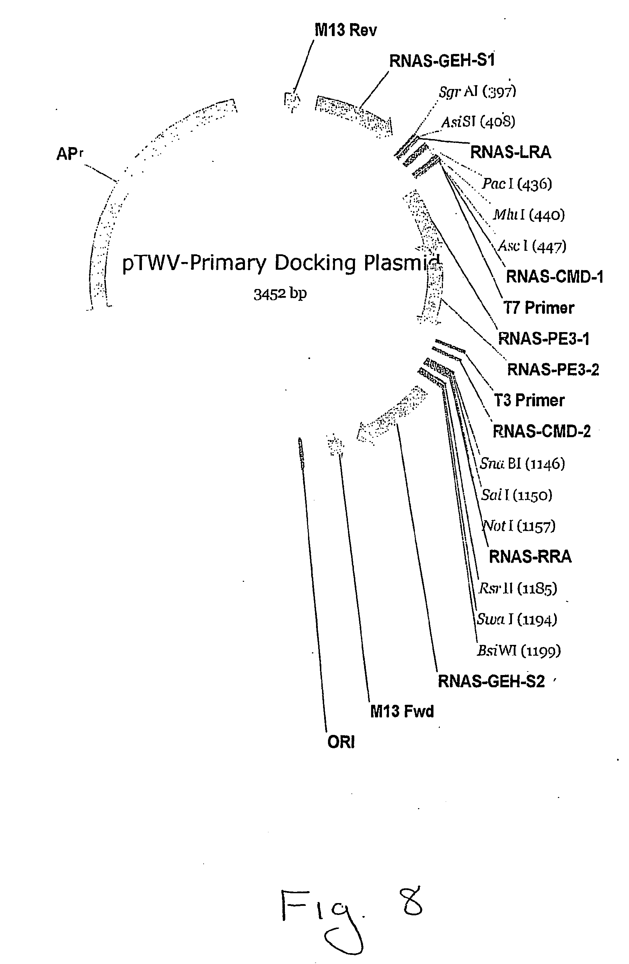

The Multigenic docking vector (Primary Docking Plasmid) shown in FIG. 8 can be used to assemble two completed PE3 transgenes that are first constructed in PE3 Docking Station Plasmids, or two homology arms needed to construct a gene-targeting transgene, or to introduce two types of positive or negative selection elements.

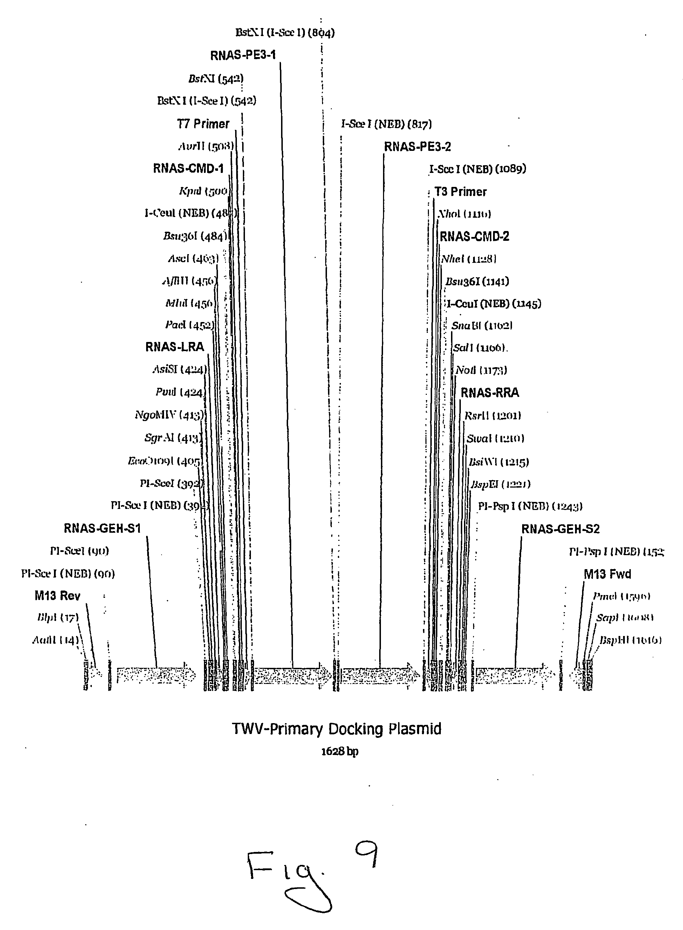

A non-limiting example of a multiple cloning site (MCS) in a Multigenic (or Primary) Docking Plasmid is shown in FIG. 9, and comprises the following sequential elements, in the order listed:
- 1.: Two non-variable and unique common restriction sites that define a 5' insertion site for the mutated pUC19 vector described above (for example, Aat II and Blp I),
- 2.: An M13 Rev. primer site,
- 3.: A pair of unique HE sites in opposite orientation flanking a random nucleotide sequence of DNA that can serve as a genome expression host selector gene acceptor module (RNAS-GEH-S1) (for example, PI-SceI (forward orientation) and PI-SceI (reverse orientation)),
- 4.: A non-variable and unique, common restriction site that allows cloning of a shuttle vector module downstream of the HE pair (for example, Eco0109I),
- 5.: A fixed grouping of non-variable rare restriction sites that define the 5' portion a Left Recombination Arm module (for example, SgrA I and AsiS I),
- 6.: Random nucleotide sequences that can serve as a Left Recombination Arm acceptor module (RNAS-LRA),
- 7.: A fixed grouping of non-variable rare restriction sites that define the 3' portion of the Left Recombination Arm acceptor module (for example, PacI, MluI, and AscI),
- 8.: A unique HE site (for example, I-Ceu I (forward orientation)),
- 9.: A pair of non-variable and unique, common restriction sites flanking a random nucleotide sequence of DNA that can serve as a chromatin modification domain acceptor module (RNAS-CMD-1) (for example, Kpn I and Avr II),
- 10.: A T7 primer site,
- 11.: A pair of unique BstX I sites in opposite orientation (wherein the variable nucleotide region in the BstX I restriction site is defined by nucleotides identical to the non-complementary tails generated by the ordering of two identical HE restriction sites arranged in reverse-complement orientation; for example, PI-SceI (forward orientation) and PI-Scel (reverse orientation)) flanking a random nucleotide sequence of DNA that can serve as a complex transgene acceptor module (RNAS-PE3-1),
- 12.: A pair of unique HE sites in opposite orientation flanking a random nucleotide sequence of DNA that can serve as a complex transgene acceptor module (RNAS-PE3-2) (for example, I-SceI (forward orientation) and I-SceI (reverse orientation)),
- 13.: A T3 primer site in reverse-orientation,
- 14.: A pair of non-variable and unique, common restriction sites flanking a random nucleotide sequence of DNA that can serve as a chromatin modification domain acceptor module (RNAS-CMD-2) (for example, Xho I and Nhe I),
- 15.: A unique HE site (here, identical to that in item 8 above (IScel), and also in reverse orientation).
- 16.: A fixed grouping of non-variable rare restriction sites that define the 5' portion a Right Recombination Arm module (for example, SnaB I, Sal I, and Not I),
- 17.: Random nucleotide sequences that can serve as a Right Recombination Arm acceptor module (RNAS-RRA),
- 18.: A fixed grouping of non-variable rare restriction sites that define the 3' portion of the Right Recombination Arm acceptor module (for example, Rsr II, Swa I, and BsiW I),
- 19.: A non-variable and unique, common restriction site that allows cloning of a shuttle vector module upstream of an HE pair (for example, BspE I),
- 20.: A pair of unique HE sites in opposite orientation flanking a random nucleotide sequence of DNA that can serve as a genome expression host selector gene acceptor module (RNAS-GEH-S2) (for example, PI-Psp I (forward orientation) and PI-Psp I (reverse orientation)),
- 21.: An M13 Forward primer site placed in reverse orientation,
- 22.: Three non-variable and unique common restriction sites that define a 3' insertion site for the mutated pUC19 vector described above (for example, Pme I, Sap I, and BspH I).

As an example of the method of practicing the present invention, a transgene can be constructed containing these elements:
- 1.: Nucleotide sequences of the human promoter for surfactant protein C (SP-C),
- 2.: Sequences encoding the protein product of the mouse gene granulocyte-macrophage colony-stimulating factor-receptor beta-c (GMRβc)
- 3.: Rabbit betaglobin intron sequences, and
- 4.: SV40 poly-A signal.

The SP-C sequence contains internal BamH1 sites, and can be released from its parental plasmid only with Not1 and EcoR1. GMRβc has an internal Not1 site, and can be cut from its parental plasmid with BamH1 and Xhol. The rabbit betaglobin intron sequences can be cut out of its parental plasmid with EcoR1. The SV-40 poly-A tail can be cut from its parental plasmid with Xho1 and Sac1. Because of redundancy of several of restriction sites, none of the parental plasmids can be used to assemble all the needed fragments.

The steps used to build the desired transgene in the PE3 Docking Plasmid invention are as follows:
- 1.: Since Not1 and PspOM1 generate compatible cohesive ends, the human SP-C promoter sequences are excised with Not1 and EcoR1 and cloned into the PspOMI and EcoR1 sites of Shuttle Vector P. The product of this reaction is called pSVP-SPC
- 2.: Following propagation and recovery steps well known to those skilled in the art, the rabbit betaglobin intron sequences are cloned into the EcoR1 site of pSVP-SPC. Orientation of the intron in the resultant intermediate construct is verified by sequencing the product, called pSVP-SPC-rβG.
- 3.: The promoter and intron are excised and isolated as one contiguous fragment from pSVP-SPC-rβG using AssiS1 and Asc1. Concurrently, the PE3 Docking Plasmid is cut with AsiS1 and Asc1 in preparation for ligation with the promoter/intron segment. The promoter/intron fragment is ligated into the Docking Plasmid, propagated, and recovered.
- 4.: The Xhol site of the GMRβc fragment is filled in to create a blunt 3' end, using techniques well known to those skilled in the art. It is then cloned into the BamH1 site and the blunt-ended Pvu2 site of pSVP-SPC-rβBG. The resultant plasmid (pDP-SPC-GMRβc-rβG) is propagated and recovered.
- 5.: The final cloning step is the addition of the SV-40 Poly-A tail. The SV40-polyA fragment is cut out with Xhol and Sac1, as is the recipient vector pDS1-SPC-GMRβc-rbβG. Both pieces of DNA are gel purified and recovered. A ligation mix is prepared with a 10:1 molar ratio of SV-40polyA to pDS1-SPC- GMRβc-rβG. The ligation products are propagated and harvested.

The new plasmid, pDS1-SPC- GMRβc-rβG-pA contains all elements required for the transgene, including a unique restriction site at the 3' end with which the entire pDS1-SPC- GMRβc-rβG-pA plasmid can be linearized for transfection into eukaryotic cells or microinjection into the pronucleus of a fertilized ovum.

Regarding the HE sites, typically at least two HE restriction sites, each able to be cleaved by at least one HE restriction enzyme, are placed flanking the modular regions, for the purpose of creating a gene cassette acceptor site that cannot self-anneal. Further, if desired, it is possible but not required to place these HE sites in opposite orientation to one another. That is, because HE sites are asymmetric and non-palindromic, it is possible to generate non-complementary protruding 3' cohesive tails by placing two HE restriction sites in opposite orientation. For example, the HE I-SceI cuts its cognate restriction site as indicated by "/":
5'...TAGGGATAA/CAGGGTAAT...3'
3'...ATCCC/TATTGTCCCATTA...5'

The reverse placement of a second site within an MCS would generate two non-complementary cohesive protruding tails:
5'...TAGGGATAA CCCTA...3'
3'...ATCCC AATAGGGAT...5'

This is particularly useful when it is desired to include large transgenes into a vector. Due to the large size of the insert, it is thermodynamically more favorable for a vector to self anneal rather than accept a large insert. The presence of non-complementary tails generated by the placement of HE restriction sites provides favorable chemical forces to counteract the thermodynamic inclination for self-ligation.

Further, the asymmetric nature of most HE protruding tails also creates a powerful cloning tool when used in combination with the BstX I restriction enzyme site (5' CCANNNNN/NTGG 3'). The sequence-neutral domain of HstX I ("N" can be any nucleotide) can be used to generate compatible cohesive ends for two reverse-oriented HE protruding tails, while precluding self-annealing.
BstX I (I-Sce I Fwd.) I-Sce I Forward I-Sce I Reverse BxtX I (I-Sce I Rev.)
5'-CCAGATAA CAGGGTAAT//ATTACCCTGTTAT GTGG-3'
3'-GGTC TATTGTCCCATTA//TAATGGGAC AATACACC-5'

Among the many advantages of the present invention, it can be readily appreciated that one can rapidly assemble transgenes, typically containing Promoter, Expression, and 3'Regulatory modules, in a very short period of time, as well as quickly and easily vary or redesign a newly assembled transgene. Conventional efforts to vary an assembled transgene using known methods would usually take a year or more of laboratory time. Using the methods of the present invention, one can make desired transgenes within days or weeks, and then do the desired testing of each, thereby saving the researcher significant time and expense. Shuttles that were originally created by de novo synthesis, recombineering, and PCR terminator over-hang cloning methods can be taken and used with the docking point technology of the present invention to rapidly assemble these pre-made elements into a multitude of transgenes. The PE3 transgenes produced using the invention can be used in a single organism, or in a variety of organisms including bacteria, yeast, mice, and other eukaryotes with little or no further modification.

## Claims

1. A domain module docking vector consisting of a DNA cloning vector, comprising a multiple cloning (MC) module for sub-cloning a genetic material of interest into the MC module, the MC module comprising:
a. a first gene pivot (GP) comprising at least two non-variable restriction sites of greater than six nucleotides operable to define the 5' portion of the MC module;
b. a nucleic acid sequence comprising a multiple cloning site (MCS) comprising a plurality of restriction sites selected from common restriction sites that are unique within the domain module docking vector, to provide cloning sites for the cloning of the genetic material of interest into the MC module; and
c. a second gene pivot comprising at least two non-variable restriction sites of greater than six nucleotides operable to define the 3' portion of the MC module,
wherein the first gene pivot and the second gene pivot independently comprise at least 3, and not more than 4 non-variable restriction sites of greater than six nucleotides.

2. The domain module docking vector according to claim 1 wherein the genetic material of interest is selected from the group consisting of a Promoter domain, an Expression domain, and a 3' Regulatory domain.

3. The domain module docking vector according to claim 1 wherein the non-variable restriction sites of greater than six nucleotides are selected from the group consisting of AsiS I, Pac I, Sbf I, Fse I, Asc I, Not I, Swa I, Rsr II, Sgr AI, Flp I, Pme I, Sda I, Sgf I, Srf I, and Sse8781 I.

4. A vector comprising:
a. a first gene pivot comprising at least two non-variable restriction sites of greater than six nucleotides that upon cloning is operable to define the 5' portion of a Promoter module;
b. a first stuffer module consisting of a first nucleic acid sequence comprising stuffer, that upon cloning is replaced by the Promoter module;
c. a second gene pivot comprising at least two non-variable restriction sites of greater than six nucleotides that upon cloning is operable to define a shared junction between the 3' portion of the Promoter module and the 5' portion of an Expression module;
d. a second stuffer module consisting of a second nucleic acid sequence comprising stuffer, that upon cloning is replaced by the Expression module;
e. a third gene pivot comprising at least two non-variable restriction sites of greater than six nucleotides that upon cloning is operable to define a shared junction between the 3' portion of the Expression module and the 5' portion of a 3' Regulatory module;
f. a third stuffer module consisting of a third nucleic acid sequence comprising stuffer, that upon cloning is replaced by the 3' Regulatory module; and
g. a fourth gene pivot comprising at least two non-variable restriction sites of greater than six nucleotides that upon cloning is operable to define the 3' portion of the 3' Regulatory module,
wherein the first gene pivot and the second gene pivot independently comprise at least 3, and not more than 4 non-variable restriction sites of greater than six nucleotides.

5. The vector according to claim 4, wherein the non-variable restriction sites of greater than six nucleotides are selected from the group consisting of AsiS I, Pac I, Sbf I, Fse I, Asc I, Not I, Swa I, Rsr II, Sgr AI, Flp I, Pme I, Sda I, Sgf I, Srf I, and Sse8781 I.

## Patentansprüche

1. Ein Domänen-Modul-Docking-Vektor bestehend aus einem DNA-Klonierungsvektor, umfassend ein Modul zur multiplen Klonierung (MC-Modul) zur Subklonierung eines genetischen Materials von Interesse in das MC-Modul, das MC-Modul umfassend:
a. ein erstes Gen-Pivot (GP), das mindestens zwei nicht-variable Restriktionsstellen von mehr als sechs Nukleotiden umfasst, und befähigt ist, den 5'-Abschnitt des MC-Moduls zu definieren;
b. eine Nukleotidsequenz umfassend eine multiple Klonierungsstelle (MCS) umfassend eine Vielzahl von im Domänen-Modul-Docking-Vektor einmaligen Restriktionsstellen, ausgewählt aus den allgemein bekannten Restriktionsstellen, zur Bereitstellung von Klonierungsstellen zur Klonierung des genetischen Materials von Interesse in das MC-Modul; und
c. ein zweites Gen-Pivot (GP), das mindestens zwei nicht-variable Restriktionsstellen von mehr als sechs Nukleotiden umfasst, und befähigt ist, den 3'-Abschnitt des MC-Moduls zu definieren;
wobei das erste Gen-Pivot und das zweite Gen-Pivot unabhängig voneinander wenigstens 3 und nicht mehr als 4 nicht-variable Restriktionsstellen von mehr als sechs Nukleotiden umfassen.

2. Der Domänen-Modul-Docking-Vektor nach Anspruch 1, wobei das genetische Material von Interesse ausgewählt ist aus der Gruppe bestehend aus einer Promotor-Domäne, einer Expressions-Domäne, und einer 3'-regulatorischen Domäne.

3. Der Domänen-Modul-Docking-Vektor nach Anspruch 1, wobei die nicht-variable Restriktionsstellen von mehr als sechs Nukleotiden ausgewählt sind aus der Gruppe bestehend aus AsiS I, Pac I, Sbf 1, Fse I, Asc I, Not I, Swa I, Rsr II, Sgr Al, Flp I, Pme I, Sda I, Sgf I, Srf I, und Sse8781 I.

4. Ein Vektor umfassend:
a. ein erstes Gen-Pivot (GP), das mindestens zwei nicht-variable Restriktionsstellen von mehr als sechs Nukleotiden umfasst, und befähigt ist den 5'-Abschnitt eines Promotor-Moduls zu definieren;
b. ein erstes Stuffer-Modul mit einem ersten Nukleotidsequenz umfassenden Stuffer, welcher beim Klonieren durch das Promotor-Modul ersetzt wird;
c. ein zweites Gen-Pivot (GP), das mindestens zwei nicht-variable Restriktionsstellen von mehr als sechs Nukleotiden umfasst, und befähigt ist beim Klonieren eine gemeinsame Verbindung zwischen dem 3'-Abschnitt des Promotor-Moduls und dem 5'-Abschnitt eines Expressions-Moduls zu definieren;
d. ein zweites Stuffer-Modul mit einem zweiten Nukleotidsequenz umfassenden Stuffer, welcher beim Klonieren durch das Expressions-Modul ersetzt wird;
e. ein drittes Gen-Pivot (GP), das mindestens zwei nicht-variable Restriktionsstellen von mehr als sechs Nukleotiden umfasst, und befähigt ist beim Klonieren eine gemeinsame Verbindung zwischen dem 3'-Abschnitt des Expressions-Moduls und dem 5'-Abschnitt eines regulatorischen Moduls zu definieren;
f. ein drittes Stuffer-Modul mit einem dritten Nukleotidsequenz umfassenden Stuffer, welcher beim Klonieren durch das regulatorische Modul ersetzt wird;
g. ein viertes Gen-Pivot (GP), das mindestens zwei nicht-variable Restriktionsstellen von mehr als sechs Nukleotiden umfasst, und befähigt ist beim Klonieren den 3'-Abschnitt des regulatorischen Moduls zu definieren; wobei das erste Gen-Pivot und das zweite Gen-Pivot unabhängig voneinander wenigstens 3 und nicht mehr als 4 nicht-variable Restriktionsstellen von mehr als sechs Nukleotiden umfassen.

5. Der Vektor nach Anspruch 4, wobei die nicht-variable Restriktionsstellen von mehr als sechs Nukleotiden ausgewählt sind aus der Gruppe bestehend aus AsiS I, Pac I, Sbf I, Fse I, Asc I, Not I, Swa I, Rsr II, Sgr Al, Flp I, Pme I, Sda I, Sgf I, Srf I, und Sse8781 I.

## Revendications

1. Vecteur d'ancrage de modules de domaines consistant en un vecteur de clonage d'ADN, comprenant un module de clonage multiple (MC) pour sous-cloner du matériel génétique d'intérêt dans le module MC, le module MC comprenant :
a. un premier gène pivot (GP) comprenant au moins deux sites de restriction non variables de plus de six nucléotides qui est fonctionnel pour définir la partie 5' du module MC ;
b. une séquence d'acide nucléique comprenant un site de clonage multiple (MCS) comprenant une pluralité de sites de restriction sélectionnés parmi des sites de restriction courants qui sont uniques au sein du vecteur d'ancrage de modules de domaines, afin de fournir des sites de clonage pour le clonage du matériel génétique d'intérêt dans le module MC ; et
c. un deuxième gène pivot comprenant au moins deux sites de restriction non variables de plus de six nucléotides qui est fonctionnel pour définir la partie 3' du module MC,
où le premier gène pivot et le deuxième gène pivot comprennent indépendamment au moins 3 et pas plus de 4 sites de restriction non variables de plus de six nucléotides.

2. Vecteur d'ancrage de modules de domaines selon la revendication 1, dans lequel le matériel génétique d'intérêt est sélectionné dans le groupe consistant en un domaine de promoteur, un domaine d'expression, et un domaine régulateur en 3'.

3. Vecteur d'ancrage de modules de domaines selon la revendication 1, dans lequel les sites de restriction non variables de plus de six nucléotides sont sélectionnés dans le groupe consistant en AsiS I, Pac I, Sbf I, Fse I, Asc I, Not I, Swa I, Rsr II, Sgr AI, Flp I, Pme I, Sda I, Sgf I, Srf I, et Sse8781 I.

4. Vecteur comprenant :
a. un premier gène pivot comprenant au moins deux sites de restriction non variables de plus de six nucléotides qui, suite à un clonage, est fonctionnel pour définir la partie 5' d'un module de promoteur ;
b. un premier module de remplissage consistant en une première séquence d'acide nucléique comprenant un fragment de remplissage qui, suite à un clonage, est remplacé par le module de promoteur ;
c. un deuxième gène pivot comprenant au moins deux sites de restriction non variables de plus de six nucléotides qui, suite à un clonage, est fonctionnel pour définir une jonction partagée entre la partie 3' du module de promoteur et la partie 5' d'un module d'expression ;
d. un deuxième module de remplissage consistant en une deuxième séquence d'acide nucléique comprenant un fragment de remplissage qui, suite à un clonage, est remplacé par le module d'expression ;
e. un troisième gène pivot comprenant au moins deux sites de restriction non variables de plus de six nucléotides qui, suite à un clonage, est fonctionnel pour définir une jonction partagée entre la partie 3' du module d'expression et la partie 5' d'un module régulateur en 3' ;
f. un troisième module de remplissage consistant en une troisième séquence d'acide nucléique comprenant un fragment de remplissage qui, suite à un clonage, est remplacé par le module régulateur en 3' ; et
g. un quatrième gène pivot comprenant au moins deux sites de restriction non variables de plus de six nucléotides qui, suite à un clonage, est fonctionnel pour définir la partie 3' du module régulateur en 3',
où le premier gène pivot et le deuxième gène pivot comprennent indépendamment au moins 3 et pas plus de 4 sites de restriction non variables de plus de six nucléotides.

5. Vecteur selon la revendication 4, dans lequel les sites de restriction non variables de plus de six nucléotides sont sélectionnés dans le groupe consistant en AsiS I, Pac I, Sbf I, Fse I, Asc I, Not I, Swa I, Rsr II, Sgr AI, Flp I, Pme I, Sda I, Sgf I, Srf I, et Sse8781 I.


### SEQUENCE LISTING

<110> Genomatix, Ltd Reed, Thomas D.
<120> DNA cloning vector Plasmids and Methods for Their use
<130> 60/417,282
<140> US 10/682,764
   <141> 2003-10-09
<150> 60/417,282
   <151> 2002-10-09
<160> 10
<170> PatentIn version 3.3
<210> 1
   <211> 3452
   <212> DNA
   <213> Artificial sequence
<220>
   <223> Synthetic Construct
<220>
   <221> misc_feature
   <222> (98)..(347)
   <223> "n" is a, c, g, or t
<220>
   <221> misc_feature
   <222> (411)..(430)
   <223> "n" is a, c, g, or t
<220>
   <221> misc_feature
   <222> (530)..(779)
   <223> "n" is a, c, g, or t
<220>
   <221> misc_feature
   <222> (810)..(1059)
   <223> "n" is a, c, g, or t
<220>
   <221> misc_feature
   <222> (1163)..(1182)
   <223> "n" is a, c, g, or t
<220>
   <221> misc_feature
   <222> (1240)..(1489)
   <223> "n" is a, c, g, or t
<400> 1
   tgagcagcgg ataacaattt cacacaggaa acagctatga ccatgattac tctgtagcat      60
   ctatgtcggg tgcggagaaa gaggtaatga aatggcannn nnnnnnnnnn nnnnnnnnnn     120
   nnnnnnnnnn nnnnnnnnnn nnnnnnnnnn nnnnnnnnnn nnnnnnnnnn nnnnnnnnnn 180
   nnnnnnnnnn nnnnnnnnnn nnnnnnnnnn nnnnnnnnnn nnnnnnnnnn nnnnnnnnnn 240
   nnnnnnnnnn nnnnnnnnnn nnnnnnnnnn nnnnnnnnnn nnnnnnnnnn nnnnnnnnnn 300
   nnnnnnnnnn nnnnnnnnnn nnnnnnnnnn nnnnnnnnnn nnnnnnntgc catttcatta 360
   cctctttctc cgcacccgac atagataggc cctgcgccgg cggcgatcgc nnnnnnnnnn 420
   nnnnnnnnnn ttaattaaac gcgtggcgcg cctaactata acggtcctaa ggtagcgagg 480
   taccgctggc cctagggtaa tacgactcac tatagggcca cataagtggn nnnnnnnnnn 540
   nnnnnnnnnn nnnnnnnnnn nnnnnnnnnn nnnnnnnnnn nnnnnnnnnn nnnnnnnnnn 600
   nnnnnnnnnn nnnnnnnnnn nnnnnnnnnn nnnnnnnnnn nnnnnnnnnn nnnnnnnnnn 660
   nnnnnnnnnn nnnnnnnnnn nnnnnnnnnn nnnnnnnnnn nnnnnnnnnn nnnnnnnnnn 720
   nnnnnnnnnn nnnnnnnnnn nnnnnnnnnn nnnnnnnnnn nnnnnnnnnn nnnnnnnnnc 780
   cacttatgtg gtagggataa cagggtaatn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnnnn nnnnnnnnna ttaccctgtt atccctatcc 1080
   ctttagtgag ggttaattct cgaggcagga gctagctcgc taccttagga ccgttatagt 1140
   tatacgtagt cgacgcggcc gcnnnnnnnn nnnnnnnnnn nncggtccga tttaaatcgt 1200
   acgtccggat ggcaaacagc tattatgggt attatgggtn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnna cccataatac 1500
   ccataatagc tgtttgccag ctacagagtt tactggccgt cgttttacaa cgtcgtgact 1560
   gggaaaaccc tggcggttta aacgctcttc cgcttccttc atgtgagcaa aaggccagca 1620
   aaaggccagg aaccgtaaaa aggccgcgtt gctggcgttt ttccataggc tccgcccccc 1680
   tgacgagcat cacaaaaatc gacgctcaag tcagaggtgg cgaaacccga caggactata 1740
   aagataccag gcgtttcccc ctggaagctc cctcgtgcgc tctcctgttc cgaccctgcc 1800
   gcttaccgga tacctgtccg cctttctccc ttcgggaagc gtggcgcttt ctcatagctc 1860
   acgctgtagg tatctcagtt cggtgtaggt cgttcgctcc aagctgggct gtgtgcacga 1920
   accccccgtt cagcccgacc gctgcgcctt atccggtaac tatcgtcttg agtccaaccc 1980
   ggtaagacac gacttatcgc cactggcagc agccactggt aacaggatta gcagagcgag 2040
   gtatgtaggc ggtgctacag agttcttgaa gtggtggcct aactacggct acactagaag 2100
   gacagtattt ggtatctgcg ctctgctgaa gccagttacc ttcggaaaaa gagttggtag 2160
   ctcttgatcc ggcaaacaaa ccaccgctgg tagcggtggt ttttttgttt gcaagcagca 2220
   gattacgcgc agaaaaaaag gatctcaaga agatcctttg atcttttcta cggggtctga 2280
   cgctcagtgg aacgaaaact cacgttaagg gattttggtc atgataacta tgactctctt 2340
   aaggtagcca aattcatgag attatcaaaa aggatcttca cctagatcct tttaaattaa 2400
   aaatgaagtt ttaaatcaat ctaaagtata tatgagtaaa cttggtctga cagttaccaa 2460
   tgcttaatca gtgaggcacc tatctcagcg atctgtctat ttcgttcatc catagttgcc 2520
   tgactccccg tggtgtagat aactacgata cgggagggct taccatctgg ccccagtgct 2580
   gcaatgatac cgcgagaccc acgctcaccg gctccagatt tatcagcaat aaaccagcca 2640
   gccggaaggg ccgagcgcag aagtggtcct gcaactttat ccgcctccat ccagtctatt 2700
   aattgttgcc gggaagctag agtaagtagt tcgccagtta atagtttgcg caacgtggtt 2760
   gccattgcta caggcatcgt ggtgtcacgc tcgtcgtttg gtatggcttc attcagctcc 2820
   ggttcccaac gatcaaggcg agttacatga tcccccatgt tgtgcaaaaa agcggttagc 2880
   tccttcggtc ctccgatcgt tgtcagaagt aagttggccg cagtgttatc actcatggtt 2940
   atggcagcac tgcataattc tcttactgtc atgccatccg taagatgctt ttctgtgact 3000
   ggtgagtact caaccaagtc attctgagaa tagtgtatgc ggcgaccgag ttgctcttgc 3060
   ccggcgtcaa tacgggataa taccgcgcca catagcagaa ctttaaaagt gctcatcatt 3120
   ggaaagcgtt cttcggggcg aaaactctca aggatcttac cgctgttgag atccagttcg 3180
   atgtaaccca ctcgtgcacc caactgatct tcagcatctt ttactttcac cagcgtttct 3240
   gggtgagcaa aaacaggaag gcaaaatgcc gcaaaaaagg gaataagggc gacacggaaa 3300
   tgttgaatac tcatactctt cctttttcaa tattattgaa gcatttatca gggttattgt 3360
   ctcatgcgcg gatacatatt tgaatgtatt tagaaaaata aacaaatagg ggttccgcgc 3420
   acatttcccc gaaaagtgcc acctgacgtc gc 3452
<210> 2
   <211> 792
   <212> DNA
   <213> Artificial sequence
<220>
   <223> PE3 Docking Plasmid MCS
<220>
   <221> misc_feature
   <222> (86)..(185)
   <223> "n" is a, c, g, or t
<220>
   <221> misc_feature
   <222> C208)..(307)
   <223> "n" is a, c, g, or t
<220>
   <221> misc_feature
   <222> (330)..(429)
   <223> "n" is a, c, g, or t
<220>
   <221> misc_feature
   <222> (451)..(550)
   <223> "n" is a, c, g, or t
<220>
   <221> misc_feature
   <222> (593)..(692)
   <223> "n" is a, c, g, or t
<400> 2
   gagagagaga cgtcgctgag caggccctgt aatacgactc actatagggg gcgccggagc 60
   ttagggataa cagggtaatg gtaccnnnnn nnnnnnnnnn nnnnnnnnnn nnnnnnnnnn 120
   nnnnnnnnnn nnnnnnnnnn nnnnnnnnnn nnnnnnnnnn nnnnnnnnnn nnnnnnnnnn 180
   nnnnncctag ggcgatcgcc gccggcgnnn nnnnnnnnnn nnnnnnnnnn nnnnnnnnnn 240
   nnnnnnnnnn nnnnnnnnnn nnnnnnnnnn nnnnnnnnnn nnnnnnnnnn nnnnnnnnnn 300
   nnnnnnntta attaaggcgc gccacgcgtn nnnnnnnnnn nnnnnnnnnn nnnnnnnnnn 360
   nnnnnnnnnn nnnnnnnnnn nnnnnnnnnn nnnnnnnnnn nnnnnnnnnn nnnnnnnnnn 420
   nnnnnnnnnt acgtagcggc cgcgtcgacg nnnnnnnnnn nnnnnnnnnn nnnnnnnnnn 480
   nnnnnnnnnn nnnnnnnnnn nnnnnnnnnn nnnnnnnnnn nnnnnnnnnn nnnnnnnnnn 540
   nnnnnnnnnn atttaaatcg gtccgcgtac gcatatagct aacagcctcg agnnnnnnnn 600
   nnnnnnnnnn nnnnnnnnnn nnnnnnnnnn nnnnnnnnnn nnnnnnnnnn nnnnnnnnnn 660
   nnnnnnnnnn nnnnnnnnnn nnnnnnnnnn nngctagcat taccctgtta tccctagccg 720
   ctggcgcttc cctttagtga gggttaattt ccggagttta aacgctcttc cgcttccttc 780
   atgagagaga ga 792
<210> 3
   <211> 3452
   <212> DNA
   <213> Artificial sequence
<220>
   <223> Primary Docking Plasmid
<220>
   <221> misc_feature
   <222> (98)..(347)
   <223> "n" is a, c, g, or t
<220>
   <221> misc_feature
   <222> (411)..(430)
   <223> "n" is a, c, g, or t
<220>
   <221> misc_feature
   <222> (530)..(779)
   <223> "n" is a, c, g, or t
<220>
   <221> misc_feature
   <222> (810)..(1059)
   <223> "n" is a, c, g, or t
<220>
   <221> misc_feature
   <222> (1163)..(1182)
   <223> "n" is a, c, g, or t
<220>
   <221> misc_feature
   <222> (1240)..(1489)
   <223> "n" is a, c, g, or t
<400> 3
   tgagcagcgg ataacaattt cacacaggaa acagctatga ccatgattac tctgtagcat      60
   ctatgtcggg tgcggagaaa gaggtaatga aatggcannn nnnnnnnnnn nnnnnnnnnn     120
   nnnnnnnnnn nnnnnnnnnn nnnnnnnnnn nnnnnnnnnn nnnnnnnnnn nnnnnnnnnn     180
   nnnnnnnnnn nnnnnnnnnn nnnnnnnnnn nnnnnnnnnn nnnnnnnnnn nnnnnnnnnn     240
   nnnnnnnnnn nnnnnnnnnn nnnnnnnnnn nnnnnnnnnn nnnnnnnnnn nnnnnnnnnn     300
   nnnnnnnnnn nnnnnnnnnn nnnnnnnnnn nnnnnnnnnn nnnnnnntgc catttcatta     360
   cctctttctc cgcacccgac atagataggc cctgcgccgg cggcgatcgc nnnnnnnnnn     420
   nnnnnnnnnn ttaattaaac gcgtggcgcg cctaactata acggtcctaa ggtagcgagg     480
   taccgctggc cctagggtaa tacgactcac tatagggcca cataagtggn nnnnnnnnnn     540
   nnnnnnnnnn nnnnnnnnnn nnnnnnnnnn nnnnnnnnnn nnnnnnnnnn nnnnnnnnnn     600
   nnnnnnnnnn nnnnnnnnnn nnnnnnnnnn nnnnnnnnnn nnnnnnnnnn nnnnnnnnnn     660
   nnnnnnnnnn nnnnnnnnnn nnnnnnnnnn nnnnnnnnnn nnnnnnnnnn nnnnnnnnnn     720
   nnnnnnnnnn nnnnnnnnnn nnnnnnnnnn nnnnnnnnnn nnnnnnnnnn nnnnnnnnnc     780
   cacttatgtg gtagggataa cagggtaatn nnnnnnnnnn nnnnnnnnnn nnnnnnnnnn     840
   nnnnnnnnnn nnnnnnnnnn nnnnnnnnnn nnnnnnnnnn nnnnnnnnnn nnnnnnnnnn     900
   nnnnnnnnnn nnnnnnnnnn nnnnnnnnnn nnnnnnnnnn nnnnnnnnnn nnnnnnnnnn     960
   nnnnnnnnnn nnnnnnnnnn nnnnnnnnnn nnnnnnnnnn nnnnnnnnnn nnnnnnnnnn    1020
   nnnnnnnnnn nnnnnnnnnn nnnnnnnnnn nnnnnnnnna ttaccctgtt atccctatcc    1080
   ctttagtgag ggttaattct cgaggcagga gctagctcgc taccttagga ccgttatagt 1140
   tatacgtagt cgacgcggcc gcnnnnnnnn nnnnnnnnnn nncggtccga tttaaatcgt 1200
   acgtccggat ggcaaacagc tattatgggt attatgggtn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnna cccataatac 1500
   ccataatagc tgtttgccag ctacagagtt tactggccgt cgttttacaa cgtcgtgact 1560
   gggaaaaccc tggcggttta aacgctcttc cgcttccttc atgtgagcaa aaggccagca 1620
   aaaggccagg aaccgtaaaa aggccgcgtt gctggcgttt ttccataggc tccgcccccc 1680
   tgacgagcat cacaaaaatc gacgctcaag tcagaggtgg cgaaacccga caggactata 1740
   aagataccag gcgtttcccc ctggaagctc cctcgtgcgc tctcctgttc cgaccctgcc 1800
   gcttaccgga tacctgtccg cctttctccc ttcgggaagc gtggcgcttt ctcatagctc 1860
   acgctgtagg tatctcagtt cggtgtaggt cgttcgctcc aagctgggct gtgtgcacga 1920
   accccccgtt cagcccgacc gctgcgcctt atccggtaac tatcgtcttg agtccaaccc 1980
   ggtaagacac gacttatcgc cactggcagc agccactggt aacaggatta gcagagcgag 2040
   gtatgtaggc ggtgctacag agttcttgaa gtggtggcct aactacggct acactagaag 2100
   gacagtattt ggtatctgcg ctctgctgaa gccagttacc ttcggaaaaa gagttggtag 2160
   ctcttgatcc ggcaaacaaa ccaccgctgg tagcggtggt ttttttgttt gcaagcagca 2220
   gattacgcgc agaaaaaaag gatctcaaga agatcctttg atcttttcta cggggtctga 2280
   cgctcagtgg aacgaaaact cacgttaagg gattttggtc atgataacta tgactctctt 2340
   aaggtagcca aattcatgag attatcaaaa aggatcttca cctagatcct tttaaattaa 2400
   aaatgaagtt ttaaatcaat ctaaagtata tatgagtaaa cttggtctga cagttaccaa 2460
   tgcttaatca gtgaggcacc tatctcagcg atctgtctat ttcgttcatc catagttgcc 2520
   tgactccccg tggtgtagat aactacgata cgggagggct taccatctgg ccccagtgct 2580
   gcaatgatac cgcgagaccc acgctcaccg gctccagatt tatcagcaat aaaccagcca 2640
   gccggaaggg ccgagcgcag aagtggtcct gcaactttat ccgcctccat ccagtctatt 2700
   aattgttgcc gggaagctag agtaagtagt tcgccagtta atagtttgcg caacgtggtt 2760
   gccattgcta caggcatcgt ggtgtcacgc tcgtcgtttg gtatggcttc attcagctcc 2820
   ggttcccaac gatcaaggcg agttacatga tcccccatgt tgtgcaaaaa agcggttagc 2880
   tccttcggtc ctccgatcgt tgtcagaagt aagttggccg cagtgttatc actcatggtt 2940
   atggcagcac tgcataattc tcttactgtc atgccatccg taagatgctt ttctgtgact   3000
   ggtgagtact caaccaagtc attctgagaa tagtgtatgc ggcgaccgag ttgctcttgc   3060
   ccggcgtcaa tacgggataa taccgcgcca catagcagaa ctttaaaagt gctcatcatt   3120
   ggaaagcgtt cttcggggcg aaaactctca aggatcttac cgctgttgag atccagttcg   3180
   atgtaaccca ctcgtgcacc caactgatct tcagcatctt ttactttcac cagcgtttct   3240
   gggtgagcaa aaacaggaag gcaaaatgcc gcaaaaaagg gaataagggc gacacggaaa   3300
   tgttgaatac tcatactctt cctttttcaa tattattgaa gcatttatca gggttattgt   3360
   ctcatgcgcg gatacatatt tgaatgtatt tagaaaaata aacaaatagg ggttccgcgc   3420
   acatttcccc gaaaagtgcc acctgacgtc gc                                 3452
<210> 4
   <211> 1628
   <212> DNA
   <213> Artificial sequence
<220>
   <223> Primary Docking Plasmid MCS
<220>
   <221> misc_feature
   <222> (114)..(363)
   <223> "n" is a, c, g, or t
<220>
   <221> misc_feature
   <222> (427)..(446)
   <223> "n" is a, c, g, or t
<220>
   <221> misc_feature
   <222> (546)..(795)
   <223> "n" is a, c, g, or t
<220>
   <221> misc_feature
   <222> (826)..(1075)
   <223> "n" is a, c, g, or t
<220>
   <221> misc_feature
   <222> (1179)..(1198)
   <223> "n" is a, c, g, or t
<220>
   <221> misc_feature
   <222> (1256)..(1505)
   <223> "n" is a, c, g, or t
<400> 4
   gagagagaga cgtcgctgag cagcggataa caatttcaca caggaaacag ctatgaccat     60
   gattactctg tagcatctat gtcgggtgcg gagaaagagg taatgaaatg gcannnnnnn    120
   nnnnnnnnnn nnnnnnnnnn nnnnnnnnnn nnnnnnnnnn nnnnnnnnnn nnnnnnnnnn    180
   nnnnnnnnnn nnnnnnnnnn nnnnnnnnnn nnnnnnnnnn nnnnnnnnnn nnnnnnnnnn    240
   nnnnnnnnnn nnnnnnnnnn nnnnnnnnnn nnnnnnnnnn nnnnnnnnnn nnnnnnnnnn    300
   nnnnnnnnnn nnnnnnnnnn nnnnnnnnnn nnnnnnnnnn nnnnnnnnnn nnnnnnnnnn    360
   nnntgccatt tcattacctc tttctccgca cccgacatag ataggccctg cgccggcggc    420
   gatcgcnnnn nnnnnnnnnn nnnnnnttaa ttaaacgcgt ggcgcgccta actataacgg    480
   tcctaaggta gcgaggtacc gctggcccta gggtaatacg actcactata gggccacata    540
   agtggnnnnn nnnnnnnnnn nnnnnnnnnn nnnnnnnnnn nnnnnnnnnn nnnnnnnnnn    600
   nnnnnnnnnn nnnnnnnnnn nnnnnnnnnn nnnnnnnnnn nnnnnnnnnn nnnnnnnnnn    660
   nnnnnnnnnn nnnnnnnnnn nnnnnnnnnn nnnnnnnnnn nnnnnnnnnn nnnnnnnnnn    720
   nnnnnnnnnn nnnnnnnnnn nnnnnnnnnn nnnnnnnnnn nnnnnnnnnn nnnnnnnnnn    780
   nnnnnnnnnn nnnnnccact tatgtggtag ggataacagg gtaatnnnnn nnnnnnnnnn    840
   nnnnnnnnnn nnnnnnnnnn nnnnnnnnnn nnnnnnnnnn nnnnnnnnnn nnnnnnnnnn    900
   nnnnnnnnnn nnnnnnnnnn nnnnnnnnnn nnnnnnnnnn nnnnnnnnnn nnnnnnnnnn    960
   nnnnnnnnnn nnnnnnnnnn nnnnnnnnnn nnnnnnnnnn nnnnnnnnnn nnnnnnnnnn   1020
   nnnnnnnnnn nnnnnnnnnn nnnnnnnnnn nnnnnnnnnn nnnnnnnnnn nnnnnattac   1080
   cctgttatcc ctatcccttt agtgagggtt aattctcgag gcaggagcta gctcgctacc   1140
   ttaggaccgt tatagttata cgtagtcgac gcggccgcnn nnnnnnnnnn nnnnnnnncg   1200
   gtccgattta aatcgtacgt ccggatggca aacagctatt atgggtatta tgggtnnnnn   1260
   nnnnnnnnnn nnnnnnnnnn nnnnnnnnnn nnnnnnnnnn nnnnnnnnnn nnnnnnnnnn   1320
   nnnnnnnnnn nnnnnnnnnn nnnnnnnnnn nnnnnnnnnn nnnnnnnnnn nnnnnnnnnn   1380
   nnnnnnnnnn nnnnnnnnnn nnnnnnnnnn nnnnnnnnnn nnnnnnnnnn nnnnnnnnnn   1440
   nnnnnnnnnn nnnnnnnnnn nnnnnnnnnn nnnnnnnnnn nnnnnnnnnn nnnnnnnnnn   1500
   nnnnnaccca taatacccat aatagctgtt tgccagctac agagtttact ggccgtcgtt   1560
   ttacaacgtc gtgactggga aaaccctggc ggtttaaacg ctcttccgct tccttcatga   1620
   gagagaga                                                             1628
<210> 5
   <211> 2092
   <212> DNA
   <213> Artificial Sequence
<220>
   <223> SVP Plasmid
<400> 5
   tgagcgtaat acgactcact atagggaggc cctgcgatcg ccgccggcgg atatcggagc 60
   tgctgggccc agggagcttc tagaggagct ggatccgctg gagaattcgg agctggaaag 120
   cttggagctg ctctgcaggg agctgcatgc gctggcgcac agctgttaat taaggcgcgc 180
   cacgcgttcc ggattccctt tagtgagggt taattgttta aacgctcttc cgcttccttc 240
   atgtgagcaa aaggccagca aaaggccagg aaccgtaaaa aggccgcgtt gctggcgttt 300
   ttccataggc tccgcccccc tgacgagcat cacaaaaatc gacgctcaag tcagaggtgg 360
   cgaaacccga caggactata aagataccag gcgtttcccc ctggaagctc cctcgtgcgc 420
   tctcctgttc cgaccctgcc gcttaccgga tacctgtccg cctttctccc ttcgggaagc 480
   gtggcgcttt ctcatagctc acgctgtagg tatctcagtt cggtgtaggt cgttcgctcc 540
   aagctgggct gtgtgcacga accccccgtt cagcccgacc gctgcgcctt atccggtaac 600
   tatcgtcttg agtccaaccc ggtaagacac gacttatcgc cactggcagc agccactggt 660
   aacaggatta gcagagcgag gtatgtaggc ggtgctacag agttcttgaa gtggtggcct 720
   aactacggct acactagaag gacagtattt ggtatctgcg ctctgctgaa gccagttacc 780
   ttcggaaaaa gagttggtag ctcttgatcc ggcaaacaaa ccaccgctgg tagcggtggt 840
   ttttttgttt gcaagcagca gattacgcgc agaaaaaaag gatctcaaga agatcctttg 900
   atcttttcta cggggtctga cgctcagtgg aacgaaaact cacgttaagg gattttggtc 960
   atgataacta tgactctctt aaggtagcca aattcatgag attatcaaaa aggatcttca 1020
   cctagatcct tttaaattaa aaatgaagtt ttaaatcaat ctaaagtata tatgagtaaa 1080
   cttggtctga cagttaccaa tgcttaatca gtgaggcacc tatctcagcg atctgtctat 1140
   ttcgttcatc catagttgcc tgactccccg tggtgtagat aactacgata cgggagggct 1200
   taccatctgg ccccagtgct gcaatgatac cgcgagaccc acgctcaccg gctccagatt 1260
   tatcagcaat aaaccagcca gccggaaggg ccgagcgcag aagtggtcct gcaactttat 1320
   ccgcctccat ccagtctatt aattgttgcc gggaagctag agtaagtagt tcgccagtta 1380
   atagtttgcg caacgtggtt gccattgcta caggcatcgt ggtgtcacgc tcgtcgtttg 1440
   gtatggcttc attcagctcc ggttcccaac gatcaaggcg agttacatga tcccccatgt 1500
   tgtgcaaaaa agcggttagc tccttcggtc ctccgatcgt tgtcagaagt aagttggccg 1560
   cagtgttatc actcatggtt atggcagcac tgcataattc tcttactgtc atgccatccg 1620
   taagatgctt ttctgtgact ggtgagtact caaccaagtc attctgagaa tagtgtatgc 1680
   ggcgaccgag ttgctcttgc ccggcgtcaa tacgggataa taccgcgcca catagcagaa 1740
   ctttaaaagt gctcatcatt ggaaagcgtt cttcggggcg aaaactctca aggatcttac 1800
   cgctgttgag atccagttcg atgtaaccca ctcgtgcacc caactgatct tcagcatctt 1860
   ttactttcac cagcgtttct gggtgagcaa aaacaggaag gcaaaatgcc gcaaaaaagg 1920
   gaataagggc gacacggaaa tgttgaatac tcatactctt cctttttcaa tattattgaa 1980
   gcatttatca gggttattgt ctcatgcgcg gatacatatt tgaatgtatt tagaaaaata 2040
   aacaaatagg ggttccgcgc acatttcccc gaaaagtgcc acctgacgtc gc 2092
<210> 6
   <211> 234
   <212> DNA
   <213> Artificial sequence
<220>
   <223> SVP Plasmid MCS
<400> 6
   cgctgagcgt aatacgactc actataggga ggccctgcga tcgccgccgg cggatatcgg 60
   agctgctggg cccagggagc ttctagagga gctggatccg ctggagaatt cggagctgga 120
   aagcttggag ctgctctgca gggagctgca tgcgctggcg cacagctgtt aattaaggcg 180
   cgccacgcgt tccggattcc ctttagtgag ggttaattgt ttaaacgctc ttcc 234
<210> 7
   <211> 2097
   <212> DNA
   <213> Artificial sequence
<220>
   <223> SVE Plasmid
<400> 7
   tgagcgtaat acgactcact atagggaggc cctgttaatt aaggcgcgcc acgcgtgata     60
   tcggagctgc tgggcccagg gagcttctag aggagctgga tccgctggag aattcggagc    120
   tggaaagctt ggagctgctc tgcagggagc tgcatgcgct ggcgcacagc tgtacgtagc    180
   ggccgcgtcg actccggatt ccctttagtg agggttaatt gtttaaacgc tcttccgctt    240
   ccttcatgtg agcaaaaggc cagcaaaagg ccaggaaccg taaaaaggcc gcgttgctgg    300
   cgtttttcca taggctccgc ccccctgacg agcatcacaa aaatcgacgc tcaagtcaga    360
   ggtggcgaaa cccgacagga ctataaagat accaggcgtt tccccctgga agctccctcg    420
   tgcgctctcc tgttccgacc ctgccgctta ccggatacct gtccgccttt ctcccttcgg    480
   gaagcgtggc gctttctcat agctcacgct gtaggtatct cagttcggtg taggtcgttc    540
   gctccaagct gggctgtgtg cacgaacccc ccgttcagcc cgaccgctgc gccttatccg    600
   gtaactatcg tcttgagtcc aacccggtaa gacacgactt atcgccactg gcagcagcca    660
   ctggtaacag gattagcaga gcgaggtatg taggcggtgc tacagagttc ttgaagtggt    720
   ggcctaacta cggctacact agaaggacag tatttggtat ctgcgctctg ctgaagccag    780
   ttaccttcgg aaaaagagtt ggtagctctt gatccggcaa acaaaccacc gctggtagcg    840
   gtggtttttt tgtttgcaag cagcagatta cgcgcagaaa aaaggatctc caagaagatc     900
   ctttgatctt ttctacgggg tctgacgctc agtggaacga aaactcacgt taagggattt 960
   tggtcatgat aactatgact ctcttaaggt agccaaattc atgagattat caaaaaggat 1020
   cttcacctag atccttttaa attaaaaatg aagttttaaa tcaatctaaa gtatatatga 1080
   gtaaacttgg tctgacagtt accaatgctt aatcagtgag gcacctatct cagcgatctg 1140
   tctatttcgt tcatccatag ttgcctgact ccccgtggtg tagataacta cgatacggga 1200
   gggcttacca tctggcccca gtgctgcaat gataccgcga gacccacgct caccggctcc 1260
   agatttatca gcaataaacc agccagccgg aagggccgag cgcagaagtg gtcctgcaac 1320
   tttatccgcc tccatccagt ctattaattg ttgccgggaa gctagagtaa gtagttcgcc 1380
   agttaatagt ttgcgcaacg ttgttgccat tgctacaggc atcgtggtgt cacgctcgtc 1440
   gtttggtatg gcttcattca gctccggttc ccaacgatca aggcgagtta catgatcccc 1500
   catgttgtgc aaaaaagcgg ttagctcctt cggtcctccg atcgttgtca gaagtaagtt 1560
   ggccgcagtg ttatcactca tggttatggc agcactgcat aattctctta ctgtcatgcc 1620
   atccgtaaga tgcttttctg tgactggtga gtactcaacc aagtcattct gagaatagtg 1680
   tatgcggcga ccgagttgct cttgcccggc gtcaatacgg gataataccg cgccacatag 1740
   cagaacttta aaagtgctca tcattggaaa gcgttcttcg gggcgaaaac tctcaaggat 1800
   cttaccgctg ttgagatcca gttcgatgta acccactcgt gcacccaact gatcttcagc 1860
   atcttttact ttcaccagcg tttctgggtg agcaaaaaca ggaaggcaaa atgccgcaaa 1920
   aaagggaata agggcgacac ggaaatgttg aatactcata ctcttccttt ttcaatatta 1980
   ttgaagcatt tatcagggtt attgtctcat gcgcggatac atatttgaat gtatttagaa 2040
   aaataaacaa ataggggttc cgcgcacatt tccccgaaaa gtgccacctg acgtcgc 2097
<210> 8
   <211> 239
   <212> DNA
   <213> Artificial Sequence
<220>
   <223> SVE Plasmid MCS
<400> 8
   cgctgagcgt aatacgactc actataggga ggccctgtta attaaggcgc gccacgcgtg 60
   atatcggagc tgctgggccc agggagcttc tagaggagct ggatccgctg gagaattcgg 120
   agctggaaag cttggagctg ctctgcaggg agctgcatgc gctggcgcac agctgtacgt 180
   agcggccgcg tcgactccgg attcccttta gtgagggtta attgtttaaa cgctcttcc 239
<210> 9
   <211> 2096
   <212> DNA
   <213> synthetic DNA molecule
<400> 9
   tgagcgtaat acgactcact atagggaggc cctgtacgta gcggccgcgt cgacgatatc     60
   ggagctgctg ggcccaggga gcttctagag gagctggatc cgctggagaa ttcggagctg    120
   gaaagcttgg agctgctctg cagggagctg catgcgctgg cgcacagctg atttaaatcg    180
   gtccgcgtac gtccggattc cctttagtga gggttaattg tttaaacgct cttccgcttc    240
   cttcatgtga gcaaaaggcc agcaaaaggc caggaaccgt aaaaaggccg cgttgctggc    300
   gtttttccat aggctccgcc cccctgacga gcatcacaaa aatcgacgct caagtcagag    360
   gtggcgaaac ccgacaggac tataaagata ccaggcgttt ccccctggaa gctccctcgt    420
   gcgctctcct gttccgaccc tgccgcttac cggatacctg tccgcctttc tcccttcggg    480
   aagcgtggcg ctttctcata gctcacgctg taggtatctc agttcggtgt aggtcgttcg    540
   ctccaagctg ggctgtgtgc acgaaccccc cgttcagccc gaccgctgcg ccttatccgg    600
   taactatcgt cttgagtcca acccggtaag acacgactta tcgccactgg cagcagccac    660
   tggtaacagg attagcagag cgaggtatgt aggcggtgct acagagttct tgaagtggtg    720
   gcctaactac ggctacacta gaaggacagt atttggtatc tgcgctctgc tgaagccagt    780
   taccttcgga aaaagagttg gtagctcttg atccggcaaa caaaccaccg ctggtagcgg    840
   tggttttttt gtttgcaagc agcagattac gcgcagaaaa aaaggatctc aagaagatcc    900
   tttgatcttt tctacggggt ctgacgctca gtggaacgaa aactcacgtt aagggatttt    960
   ggtcatgata actatgactc tcttaaggta gccaaattca tgagattatc aaaaaggatc   1020
   ttcacctaga tccttttaaa ttaaaaatga agttttaaat caatctaaag tatatatgag   1080
   taaacttggt ctgacagtta ccaatgctta atcagtgagg cacctatctc agcgatctgt   1140
   ctatttcgtt catccatagt tgcctgactc cccgtggtgt agataactac gatacgggag   1200
   ggcttaccat ctggccccag tgctgcaatg ataccgcgag acccacgctc accggctcca   1260
   gatttatcag caataaacca gccagccgga agggccgagc gcagaagtgg tcctgcaact   1320
   ttatccgcct ccatccagtc tattaattgt tgccgggaag ctagagtaag tagttcgcca   1380
   gttaatagtt tgcgcaacgt tgttgccatt gctacaggca tcgtggtgtc acgctcgtcg   1440
   tttggtatgg cttcattcag ctccggttcc caacgatcaa ggcgagttac atgatccccc   1500
   atgttgtgca aaaaagcggt tagctccttc ggtcctccga tcgttgtcag aagtaagttg   1560
   gccgcagtgt tatcactcat ggttatggca gcactgcata attctcttac tgtcatgcca   1620
   tccgtaagat gcttttctgt gactggtgag tactcaacca agtcattctg agaatagtgt   1680
   atgcggcgac cgagttgctc ttgcccggcg tcaatacggg ataataccgc gccacatagc   1740
   agaactttaa aagtgctcat cattggaaag cgttcttcgg ggcgaaaact ctcaaggatc   1800
   ttaccgctgt tgagatccag ttcgatgtaa cccactcgtg cacccaactg atcttcagca 1860
   tcttttactt tcaccagcgt ttctgggtga gcaaaaacag gaaggcaaaa tgccgcaaaa 1920
   aagggaataa gggcgacacg gaaatgttga atactcatac tcttcctttt tcaatattat 1980
   tgaagcattt atcagggtta ttgtctcatg cgcggataca tatttgaatg tatttagaaa 2040
   aataaacaaa taggggttcc gcgcacattt ccccgaaaag tgccacctga cgtcgc 2096
<210> 10
   <211> 238
   <212> DNA
   <213> synthetic DNA molecule
<400> 10
   cgctgagcgt aatacgactc actataggga ggccctgtac gtagcggccg cgtcgacgat 60
   atcggagctg ctgggcccag ggagcttcta gaggagctgg atccgctgga gaattcggag 120
   ctggaaagct tggagctgct ctgcagggag ctgcatgcgc tggcgcacag ctgatttaaa 180
   tcggtccgcg tacgtccgga ttccctttag tgagggttaa ttgtttaaac gctcttcc 238